# EUROPEAN PATENT APPLICATION

(11) **EP 2 431 385 A2**
(43) Date of publication of application: **21.03.2012**
(21) Application number: 10178613.5
(22) Date of filing: 02.06.2003
(51) Int. Cl.: C07K 14/435, A61K 38/17, A61K 31/175, A61K 31/4188

(54) **Combination therapy with chlorotoxin**

(30) Priority: 31.05.2002 US 384171 P; 27.08.2002 US 406033 P
(62) Divisional of application: 09176234.4
(71) Applicant: Transmolecular, inc., King of Prussia Pennsylvania 19406 (US)
(72) Inventor: Alvarez, Vernon L., Morrisville, PA 19067 (US); Grimes, Carol A., Hoover, AL 35244 (US); Gonda, Matthew A., Birmingham, AL 35242 (US)
(74) Representative: Lee, Nicholas John

(57) **Abstract**

This invention includes compositions for combination therapy, particularly involving at least one chemotherapeutic agent used in combination with chlorotoxin or a derivative thereof.

## Description

### Related Applications

This application claims the benefit of U.S. Provisional Application 60/406,033 (filed August 27, 2002) and U,S. Provisional Application 60/384,171 (filed May 31, 2002) both of which are hereby incorporated by reference in their entirety.

### Field Of The Invention

The present invention relates generally to the fields of cell physiology and oncology. More specifically, the present invention relates to a novel method of treating cell proliferative disorders, such as cancers, with doses of chlorotoxin and/or derivatives thereof in combination with chemotherapeutic agents.

### Background of the invention

Tumors that originate in brain tissue are known as primary brain tumors as opposed to secondary brain tumors that develop when cancer metastatizes to the brain. Primary brain tumors are classified by the type of tissue in which they begin. The most common brain tumors are gliomas, which begin in the glial (supportive) tissue. Astrocytomas are a type of glioma that arise from small, stix-shaped cells called astrocytes. They may grow anywhere in the brain or spinal cord but most often arise in the cerebrum in adults and the brain stem, the cerebrum, and the cerebellum in children. A grade III astrocytoma is sometimes called anaplastic astrocytoma while a grade IV astrocytoma is usually called glioblastoma multiforme. Brain stem gliomas occur in the lowest, stemlike part of the brain. Tumors in this area generally cannot be removed. Most brain stem gliomas are high-grade astrocytomas. Ependymomas are a type of glioma that usually develop in the lining of the ventricles and may also occur in the spinal cord. Although these tumors can develop at any age, they are most common in childhood and adolescence. Oligodendrogliomas arise in the cells that produce myelin, the fatty covering that protects nerves, These rare tumors usually arise in the cerebrum, grow slowly, usually do not spread into surrounding brain tissue and occur most often in middle-aged adults but have been found in people of all ages.

There are other types of brain tumors that do not originate in glial tissue. Medulloblastomas were once thought to develop from glial cells. However, recent research suggests that these tumors develop from primitive (developing) nerve cells that normally do not remain in the body after birth. For this reason, medulloblastomas are sometimes called primitive neuroectodermal tumors. Most medulloblastomas arise the cerebellum, however, they may occur in other areas as well. Meningiomas grow from the meninges and are usually benign. Because these tumors grow very slowly, the brain may be able to adjust to their presence and therefore these tumors often grow quite large before they cause symptoms. Schwannomas are benign tumors that begin in Schwann cells, which produce the myelin that protects the acoustic nerve. Acoustic neuromas are a type of schwannoma and occur mainly in adults. Craniopharyngiomas develop in the region of the pituitary gland near the hypothalamus and are usually benign, however, they are sometimes considered malignant because they can press on or damage the hypothalamus and affect vital functions. Germ cell tumors arise from primitive (developing) sex cells or germ cells. The most frequent type of germ cell tumor in the brain is the germinoma. Pineal region tumors occur in or around the pineal gland, a tiny organ near the center of the brain. The tumor can be slow growing (pineocytoma) or fast growing (pineoblastoma). The pineal region is very difficult to reach, and these tumors often cannot be removed.

Primitive neuroectodermal tumors are found both in the central and peripheral nervous systems. Primitive neuroectodermal tumors found only in the peripheral nervous system are referred to as peripheral primitive neuroectodermal tumors. Primitive neuroectodermal tumors manifest preferentially in children and have capacity for developing into a variety of neuronal, astrocytic, ependymal, muscular and melanotic lines. The conceptual basis of grouping these tumors together is based upon sharing common progenitor cells as well as sharing similar neoplastic transformations leading to tumors of similar morphological features and biological behavior. However, there remains controversy in placing all primitive neuroectodermal tumors into the same categories.

Supratentorial primitive neuroectodermal tumors include cerebral medulloblastomas, cerebral neuroblastomas, ependymoblastoma and other primitive neuroectodermal tumors, such as pineoblastomas. Peripheral neuroblastic tumors of the adrenal gland (medulla) and sympathetic nervous system are the most common type of childhood tumor outside of the central nervous system. Primary sites for these primitive neuroectodermal tumors are in the adrenals, abdominal, thoracic, cervical and pelvic sympathetic ganglia but include other primary sites as orbit, kidney, lung, skin, ovary, spermatic cord, and urinary Madder. Specific names of these related tumors are pheochromocytomas, paraganglioma, neuroblastomas, ganglioneuromas, ganglioneuroblastomas, neurofibromas, schwannomas, and malignant peripheral nerve sheath tumors. These all share common origin in the neural crest. Medulloblastomas are members of the primitive neuroectodermal tumors that are described as highly malignant embryonal tumors of the central nervous system found in the cerebellum.

Currently, surgery is the treatment of choice for tumors of the central nervous system. Surgery provides a definite diagnosis, relieves the mass bulkiness of the tumor and extends survival of the patient. The only post-surgery adjuvant treatment which is known to work effectively on central nervous system tumors is radiation, and it can prolong survival. Radiation treatment, however, has many undesirable side effects. It can damage the normal tissue of the patient, including the neuronal tissue. Radiation also can cause severe side effects (*e*.*g*., nausea, vomiting, hair loss).

The other common post-surgery adjuvant cancer treatment, chemotherapy, is relatively ineffective against neuroectodermal tumors. For example, chemotherapy against neuroectodermal tumors with nitrosourea agents is not curative. Many other cancer treating agents have been studied and tested, but generally they have a minimal effect on extending survival because many agents do not cross the blood-brain barrier. In view of these limited treatment options, the current prognosis for patients diagnosed with neuroectodermal tumors is not favorable. The median survival term for patients diagnosed with malignant astrocytomas having surgery and no adjuvant treatment is about fourteen weeks. Radiation therapy after surgery extends the median to about thirty-six weeks. The current two year survival rate for all forms of treatment is less than ten percent.

Other types of tumors are also difficult to combat by known cancer treatments. Lung cancer kills more Americans annually than the next four most frequently diagnosed neoplasms combined (Greenlee et al. (2001) CA Cancer J. Clin. 51,15-36). Approximately eighty percent of primary lung tumors are of the non-small cell variety, which includes squamous cell and large cell carcinomas, a.s well as adenocarcinomas. Single-modality therapy is considered appropriate for most cases of early and late stage non-small cell lung cancer. Early stage tumors are potentially curable with surgery, chemotherapy, or radiotherapy, and late stage patients usually receive chemotherapy or best supportive care. Intermediate stage or locally advanced non-small cell lung cancer, which comprises twenty-five to thirty-five percent of all cases, is more typically treated with multi-modality therapy.

Breast cancer also presents treatment difficulties using known The incidence of breast cancer in the United States has been rising at a rate of about two percent per year since 1980, and the American Cancer Society estimated that 192,000 cases of invasive breast cancer were diagnosed in 2001. Breast cancer is usually treated with surgery, radiotherapy, chemotherapy, hormone therapy or combinations of the various methods. A major reason for the failure of cancer chemotherapy in breast cancer is the development of resistance to the cytotoxic drugs. Combination therapy using drugs with different mechanisms of action is an accepted method of treatment which prevents development of resistance by the treated tumor. Anti-angiogenic agents are particularly useful in combination therapy because they are not likely to cause resistance development since they do not act on the tumor, but on normal host tissue.

Compositions (see U.S. patent 5,905,027) and methods (see U.S. patent 6,028,174) for diagnosing and treating neuroectodermal tumors (*e*.*g*., gliomas and meningiomas) have been developed based on the ability of chlorotoxin to bind to tumor cells of neuroectodermal origin (Soroceanu et al. (1998) Cancer Res. 58, 4871-4879; Ullrich et al. (1996) Neuroreport 7,1020-1024; Ullrich et al. (1996) Am. J. Physiol. 270, C1511-C1521). Diagnosis of neuroectodermal tumors is accomplished by identification of labeled chlorotoxin bound to tumor cells while treatment of neuroectodermal tumors is accomplished by targeting tumors with cytotoxic agents linked to chlorotoxin. Chlorotoxin is a thirty-six amino acid protein naturally derived from *leiurus quinquestriatus* scorpion venom (DeBin et al. (1993) Am. J. Physiol. 264, C361-369). The present invention expands this area of therapeutics by providing a method for treating cancer using chlorotoxin, in combination with other, conventional cancer treating agents.

### Summary of the Invention

The invention includes methods for treating cancer comprising administering chlorotoxin or a chlorotoxin derivative in combination with at least one chemotherapeutic agent. In some embodiments chlorotoxin or a chlorotoxin derivative is administered prior to administration of the chemotherapeutic agent, while in other embodiments it is administered simultaneously with the chemotherapeutic agent while in yet other embodiments, it is administered subsequent to the chemotherapeutic agent.

In another embodiment of the methods of the invention, the chemotherapeutic agent is selected from the group consisting of alkylating agents, purine antagonists, pyrimidine antagonists, plant alkaloids, intercalating antibiotics, aromatase inhibitors, anti-metabolites, mitotic inhibitors, growth factor inhibitors, cell cycle inhibitors, enzymes, topoisomerase inhibitors, biological response modifiers, anti-hormones and anti-androgens. Examples of such chemotherapeutic agents to be used in the methods of the invention include, but are not limited to, BCNU, cisplatin, gemcitabine, hydroxyurea, paclitaxel, temozomide, topotecan, fluorouracil, vincristine, vinblastine, procarbazine, dacarbazine, altretamine, cisplatin, methotrexate, mercaptopurine, thioguanine, fludarabine phosphate, cladribine, pentostatin, fluorouracil, cytarabine, azacitidine, vinblastine, vincristine, etoposide, teniposide, irinotecan, docetaxel, doxorubicin, daunorubicin, dactinomycin, idarubicin, plicamycin, mitomycin, bleomycin, tamoxifen, flutamide, leuprolide, goserelin, aminoglutethimide, anastrozole, amsacrine, asparaginase, mitoxantrone, mitotane and amifostine.

In yet another embodiment, the methods of the invention are useful for treating types of cancer selected from the group consisting of lung cancer, bone cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), neuroectodermal cancer, spinal axis tumors, glioma, meningioma and pituitary adenoma.

The invention also includes compositions for treating cancer comprising chlorotoxin or a chlorotoxin derivative and at least one chemotherapeutic agent. In some embodiments, the chemotherapeutic agent is selected from the group consisting of alkylating agents, purine antagonists, pyrimidine antagonists, plant alkaloids, intercalating antibiotics, aromatase inhibitors, anti-metabolites, mitotic inhibitors, growth factor inhibitors, cell cycle inhibitors, enzymes, topoisomerase inhibitors, biological response modifiers, anti-hormones and anti-androgens. Examples of such chemotherapeutic agents to be used in the compositions of the invention include, but are not limited to, BCNU, cisplatin, gemcitabine, hydroxyurea, paclitaxel, temozomide, topotecan, fluorouracil, vincristine, vinblastine, procarbazine, dacarbazine, altretamine, cisplatin, methotrexate, mercaptopurine, thioguanine, fludarabine phosphate, cladribine, pentostatin, fluorouracil, cytarabine, azacitidine, vinblastine, vincristine, etoposide, teniposide, irinotecan, docetaxel, doxorubicin, daunorubicin, dactinomycin, idarubicin, plicamycin, mitomycin, bleomycin, tamoxifen, flutamide, leuprolide, goserelin, aminoglutethimide, anastrozole, amsacrine, asparaginase, mitoxantrone, mitotane and amifostine.

The compositions of the invention are useful for treatment of one or more types of cancer selected from the group consisting of lung cancer, bone cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), neuroectodermal cancer, spinal axis tumors, glioma, meningioma and pituitary adenoma.

The invention also includes methods for detecting the presence of cancer in a patient comprising administering a detectable amount of labeled chlorotoxin or a chlorotoxin derivative, including radiolabeled chlorotoxin or a derivative thereof. Acceptable radiolabels include, but are not limited to, ³H, ¹⁴C, ¹⁸F, ¹⁹F, ³¹P, ³²P, ³⁵S, ¹³¹I, ¹²⁵I, ¹²³I, ⁶⁴Cu, ¹⁸⁷Re, ¹¹¹In, ⁹⁰Y, ^{99m}Tc and ¹⁷⁷Lu. Types of detectable cancer include, but are not limited to, lung cancer, bone cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), neuroectodermal cancer, spinal axis tumors, glioma, meningioma and pituitary adenoma.

### Brief Description of Figures

Figure 1 depicts the effect of temodar in combination with chlorotoxin *in vitro*. D54 glioma cells were incubated with saline alone (control), temodar alone, temodar plus chlorotoxin, or pretreated with chlorotoxin twenty-four hours prior to temodar treatment.
Figure 2 depicts the effect of chlorotoxin on temodar efficacy *in vivo*. Nude mice with established U251 glioma flask tumors were treated with saline alone (control), temodar alone, or temodar plus chlorotoxin.
Figure 3 depicts the effect of chlorotoxin pretreatment on hydroxyurea efficacy *in vivo*. Nude mice with established D54 glioma flank tumors were treated with saline alone (control), hydroxyurea alone, or chlorotoxin plus hydroxyurea.
Figure 4 depicts a cytotoxicity assay in which low concentrations of chlorotoxin are shown to inhibit the growth and proliferation of glioblastoma cells.
Figure 5 depicts the effect of four day incubation and wash-out on the ability of chlorotoxin to inhibit abnormal cell growth.
Figure 6 depicts a cytotoxicity assay in which low concentrations of chlorotoxin are shown to inhibit the growth and proliferation of prostate cancer cells.
Figure 7 depicts an *in vivo* assay of the ability of chlorotoxin to inhibit the growth of glioblastoma tumor cells in athymic nude mice.
Figure 8 depicts an *in vivo* assay of the ability of chlorotoxin to enhance survival of athymic nude mice with intracranial glioblastoma tumors. Cessation of intravenous treatment indicated by arrow.
Figure 9 depicts an *in vivo* assay of the ability of chlorotoxin to inhibit growth of glioblastoma tumors in the flanks of athymic nude mice.
Figure 10 depicts a series of overlapping 10-mer peptides derived from chlorotoxin. Cysteine residues of SEQ ID NO: 1 are replaced in the 10-mers with serine to prevent cross-linking of peptides.
Figure 11 depicts binding of chlorotoxin and 10-mer peptides 1-15.
Figure 12 depicts binding of chlorotoxin and 10-mer peptides 16-27, 1, 5 and 10.
Figure 13 depicts binding of peptide 21, the native core 9-mer, and each alanine-substituted 9-mer peptide to both U251 and PC3 cells.
Figure 14 depicts binding of short scorpion toxins in PC3 human prostate cancer cells.
Figure 15 depictss the effect of peptide 21 on the proliferation of D54MG cells was studied by adding increasing doses of peptide 21 to the cells and then measuring the uptake of ³H-thymidine.

### Detailed Description

This invention relates to combination chemotherapy, particularly involving at least one chemotherapeutic agent used in combination with chlorotoxin or a derivative thereof. In one aspect, the invention includes compositions and methods for killing a cancer cell by first administering to a cancer cell chlorotoxin in combination with a chemotherapeutic agent. The present invention includes a method of retarding the growth of a tumor by administering chlorotoxin to tumor simultaneously with the chemotherapeutic agent. In another aspect, the invention includes compositions and methods for killing a cancer cell or retarding the growth of a tumor by first administering chlorotoxin (or a chlorotoxin derivative) and subsequently administering a chemotherapeutic agent. The present invention also includes a method of killing a cancer cell or retarding the growth of a tumor by first administering a chemotherapeutic agent and subsequently administering chlorotoxin (or a chlorotoxin derivative). The prior, simultaneous or subsequent administration of chlorotoxin or a derivative thereof may also have the effect of reducing the amount of chemotherapeutic agent necessary for successful treatment thus reducing the severe side effects associated with chemotherapeutic agents.

### Combination Chemotherapeutic Compositions

This invention includes a pharmaceutical composition for the treatment of abnormal cell growth in a mammal, including a human, comprising an amount of chlorotoxin or a chlorotoxin derivative, in combination, with an chemotherapeutic agent, that is effective in enhancing the effects of the chemotherapeutic agent in inhibiting abnormal cell growth (*i*.*e*., acts as an adjuvant for the chemotherapeutic agent) and a pharmaceutically acceptable carrier. As used herein, the term "abnormal gell growth" unless otherwise indicated, refers to cell growth that is independent of normal regulatory mechanisms (*e*.*g*., loss of contact inhibition). This includes the abnormal growth and/or proliferation of cells in both benign and malignant cells of neoplastic diseases. Chemotherapeutic-dependent inhibition of abnormal cell growth can occur by a variety of mechanisms including, but not limited to, cell death, apoptosis, inhibition of cell division, transcription, translation, transduction, etc.

In one embodiment of said composition, the abnormal cell growth is cancer. As used herein, the term "cancer" unless otherwise indicated, refers to diseases that are characterized by uncontrolled, abnormal cell growth and/or proliferation. Types of cancer where the composition is useful include, but are not limited to, lung cancer, bone cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), neuroectodermal cancers, spinal axis tumors, glioma, meningioma, pituitary adenoma, or a combination of one or more of the foregoing cancers. In another embodiment of said pharmaceutical composition, said abnormal cell growth is a benign proliferative disease, including, but not limited to, benign prostatic hyperplasia, hypertrophy or restinosis.

As discussed above, the invention includes a pharmaceutical composition for the treatment of abnormal cell growth in a mammal, including a human, which comprises an amount of a chlorotoxin, as defined above, in combination with at least one chemotherapeutic agent and a pharmaceutically acceptable carrier. As used herein, the term "chemotherapeutic agent" unless otherwise indicated, refers to any agent used in the treatment of cancer which inhibits, disrupts, prevents or interferes with abnormal cell growth and/or proliferation. Examples of chemotherapeutic agents include, but are not limited to, alkylating agents, purine antagonists, pyrimidine antagonists, plant alkaloids, intercalating antibiotics, aromatase inhibitors, anti-metabolites, mitotic inhibitors, growth factor inhibitors, cell cycle inhibitors, enzymes, topoisomerase inhibitors, biological response modifiers, steroid hormones and anti-androgens. In some embodiments, chlorotoxin or a derivative thereof can be combined with a single species of chemotherapeutic agent while in other embodiments, chlorotoxin can be combined with multiple species of chemotherapeutic agents.

Examples of alkylating agents include, but are not limited to, carmustine, lomustine, cyclophosphamide, ifosfamide, mechlorethamine and streptozotocin. Examples of antibiotics include, but are not limited to, bleomycin, dactinomycin, daunorubicin, doxorubicin, idarubicin and plicamycin. Examples of anti-metabolites include, but are not limited to, cytarabine, fludarabine, 5-fluorouracil, 6-mercaptopurine, methotrexate and 6-thioguanine. Examples of mitotic inhibitors include, but are not limited to, navelbine, paclitaxel, vinblastine and vincristine, Examples of steroid hormones and anti-androgens include, but are not limited to, aminoglutethimides, estrogens, flutamide, goserelin, leuprolide, prednisone and tamoxifen.

In some aspects, the invention includes a population of conjugate molecules, said conjugate molecules comprising at least one chlorotoxin peptide or a derivative thereof and at least one chemotherapeutic agent, wherein the extent of conjugation of chlorotoxin and the chemotherapeutic agent is such that the effect of the chemotherapeutic agent in a mammal receiving the conjugate may be enhanced when compared to mixtures of the chemotherapeutic agent with chlorotoxin, or the chemotherapeutic agent alone. In another aspect, the invention includes compositions comprising a population of conjugate molecules wherein at least one chlorotoxin peptide or derivative thereof is conjugated to at least one chemotherapeutic agent and a pharmaceutically acceptable excipient. In some embodiments, chlorotoxin or a derivative thereof can be conjugated to a single species of chemotherapeutic agent while in other embodiments, chlorotoxin can be conjugated to multiple species of chemotherapeutic agents.

As used herein, the term "chlorotoxin" unless otherwise described, refers to the full-length, thirty-six amino acid polypeptide naturally derived from *Leiurus quinquestriatus* scorpion venom (DeBin et al. (1993) Am. J. Physiol. 264, C361-369) which comprises the amino acid sequence of native chlorotoxin as set forth in SEQ ID NO: 1. The term "chlorotoxin" includes polypeptides comprising SEQ ID NO: 1 which have been synthetically or recombinantly produced, such as those disclosed in U.S. Patent 6,319,891, which is herein incorporated by reference in its entirety.

As used herein, the term "chlorotoxin subunit" or "subunit of chlorotoxin" refers to a peptide comprising less than thirty-six contiguous amino acids of chlorotoxin and which is capable of specifically binding to cancer cells.

As used herein, the term "chlorotoxin derivative" refers to derivatives, analogs, variants, polypeptide fragments and mimetics of chlorotoxin and related peptides which retain the same activity as chlorotoxin, such as binding specifically binding to a cancer cell when compared to a normal cell, can also be used for practicing the methods of the invention, Examples of derivatives include, but are not limited to, peptide variants of chlorotoxin, peptide fragments of chlorotoxin, for example, fragment comprising or consisting of contiguous 10-mer peptides of SEQ ID NO: 1, 2, 3, 4, 5, 6 or 7 or comprising about residues 10-18 or 21-30 of SEQ ID NO: 1, core binding sequences, and peptide mimetics.

Chlorotoxin and peptide derivatives thereof can be prepared using standard solid phase (or solution phase) peptide synthesis methods, as is known in the art. In addition, the nucleic acids encoding these peptides may be synthesized using commercially available oligonucleotide synthesis instrumentation and produced recombinantly using standard recombinant production systems. The production using solid phase peptide synthesis is necessitated if non-gene-encoded amino acids are to be included. The term "chlorotoxin derivative" as used herein is synonymous with "variant" also includes modifications to the chlorotoxin sequence by one or more deletions of up to 10 (*e*.*g*., 1 to 7 or 1 to 5 amino acids; insertions of a total of up to 10 (*e*.*g*., 1 to 5) amino acids internally within the amino acid sequence of chlorotoxin; or of up to a total of 100 amino acids at either terminus of the chlorotoxin sequence; or conservative substitutions of a total of up to 15 (*e*.*g*., to 5) amino acids.

Derivatives of chlorotoxin include polypeptides comprising a conservative or non-conservative substitution of at least one amino acid residue when the derivative sequence and the chlorotoxin sequence are maximally aligned. The substitution may be one which enhances at least one property or function of chlorotoxin, inhibits at least one property or function of chlorotoxin, or is neutral to at least one property or function of chlorotoxin. As used herein, a "property or function" of chlorotoxin includes, but is not limited to, at least one selected from the group consisting of the ability to arrest abnormal cell growth, cause paralysis of a subject, specific binding to a benign or malignant cancer cell when compared to a non-cancer cell (*i*.*e*., normal), and killing of a benign or malignant cancer cell. In terms of the present disclosure, the cancer cell may be *in vivo*, *ex vivo*, *in vitro*, a primary isolate from a subject, a cultured cell or a cell line.

Derivatives of chlorotoxin further include polypeptide comprising the amino acid sequence KGRGKSY (SEQ ID NO: 8), corresponding to amino acid residues 23-29 of SEQ ID NO: 1. Derivatives of chlorotoxin also include polypeptides comprising the amino acid sequence TTX₁X₂X₃MX₄X₅K (SEQ ID NO: 13) corresponding to amino acid residues 7-15 of SEQ ID NO: 1, wherein X₁ is an acidic amino acid selected from the group consisting of aspartic acid and glutamic acid; X₂ is an amino acid selected from the group consisting of alanine, arginine, asparagine, aspartic acid, cysteine, glutamine, glutamic acid, glycine, histidine, isoleucine, leucine, lysine, proline, methionine, phenylalanine, serine, threonine, tryptophan, tyrosine and valine; X₃ is an amide amino acid selected from the group consisting of asparagine and glutamine; X₄ is an any amino acid but in a preferred embodiment is selected from the group consisting of serine, threonine and alanine; and X₅ is a basic amino acid selected from the group consisting of histine, lysine and arginine. In one embodiment, X₁ is aspartic acid, X₂ is histidine or proline, X₃ is glutamine, X₄ is alanine and X₅ is arginine or lysine.

Peptide variants of chlorotoxin include, but are not limited to, deletion or conservative amino acid substitution variants of SEQ ID NO: 1. As used herein, a conservative variant refers to alterations in the amino acid sequence that do not adversely substantially affect the biological functions of the peptide. A substitution, insertion or deletion is said to adversely affect the peptide when the altered sequence substantially prevents or disrupts a biological function associated with the peptide (*e.g.,* binding to a cancer cell). For example, the overall charge, structure or hydrophobic/hydrophilic properties of the peptide can be altered without adversely affecting a biological activity. Accordingly, the amino acid sequence can be altered, for example to render the peptide more hydrophobic or hydrophilic, without adversely affecting the biological activities of the peptide.

The methods of the invention include corresponding polypeptide toxins of other scorpion species that display similar or related activity to chlorotoxin for the diagnosis and treatment of diseases associated with abnormal cell proliferation as described herein, including cancer. For purposes of the specification, "similar or related activity to chlorotoxin" is defined as binding to cells displaying abnormal cell growth, including benign cells exhibiting abnormal growth and malignant cancer cells. Examples of such polypeptide toxins include, but are not limited to, toxins which contain one or more of the binding domains of chlorotoxin set forth in SEQ ID NO: 8 or SEQ ID NO: 13, and any of the consensus sequences set forth in Table 1.

**Table 1 - Scorpion toxin alignments (sequence identifier in parenthesis)**

| | | |
|---|---|---|
| Small Toxin (Peptide 1) - *Mesobuthus tamulus sindicus* | | |
| Chlorotoxin (SEQ | ID NO: 1) | 1                                  36 MCMPCFTTDHQMARKCDDCCGGKGRGKCYGPQCLCR RCKPCFTTDPQMSKKCADCCGGKGKGKCYGPQCLC- C PCFTTD QMAKKC DCCGGKGKGKCYGPQCLC |
| Small Toxin | (SEQ ID NO: 15) | |
| Consensus | (SEQ ID NO: 16) | |
| Probable Toxin LQH 8/6 *- Leiurus quinquestriatus hebraeus* | | |
| Chlorotoxin | (SEQ ID NO: 1) | 1                                    38 MCMPCFTTDHQMARKCDDCCGGKGRGKCYGPQCLCR-- RCSPCFTTDQQMTKKCYDCCGGKGKGKCYGPQCICAPY C PCFTTD QM KKC DCCGGKGKGKCYGPQCIC |
| Toxin LQH | (SEQ ID NO: 17) | |
| Consensus | (SEQ ID NO: 18) | |
| Chinese Scorpion - *Mesobuthus martensii* | | |
| Chlorotoxin | (SEQ ID NO: 1) | 1                                 35 -----------------------MCMPCFTTDHQM MKFLYGIVFIALFLTVMFATQTDGCGPCFTTDANM C PCFTTD NM |
| Chinese | (SEQ ID NO: 19) | |
| Consensus | (SEQ ID NO: 20) | |
| Chlorotoxin | (SEQ ID NO: 1) | 36                                                61 ARKCDDCCGGKGRGKCYGPQCLCR-- ARKCRECCGGIG--KCFGPQCLCNRI ARKC DCCGG G  KCFGPQCLC |
| Chinese | (SEQ ID NO: 19) | |
| Consensus | (SEQ ID NO: 20) | |
| Chlorotoxin | (SEQ ID NO: 1) | 1                                 35 -----------------------MCMPCFTTDHNM MKFLYGIVFIALFLTVMFATQTDGCGPCFTTDANM C PCFTTD NM |
| Chinese | (SEQ ID NO 21) | |
| Consensus | (SEQ ID NO 22) | |
| Chlorotoxin | (SEQ ID NO 1) | 36                    59 ARKCDDCCGGKGRGKCYGPQCLCR ARKCRECCGGIGKCFGPQCLCNRI ARKC DCCGG GK    C |
| Chinese | (SEQ ID NO 21) | |
| Consensus | (SEQ ID NO 22) | |
| Insect toxin 15 (lesser Asian Scorpion) - *Mesobuthus eupeus* | | |
| Chlorotoxin | (SEQ ID NO: 1) | 1                                    37 MCMPCFTTDHQMARKCDDCCGGKGRGKCYGPQCLCR- MCMPCFTTDPNMANKCRDCCGG-GK-KCFGPQCLCNR MCMPCFTTD NMA KC DCCGG GK KCFGPQCLC 1                                   36 MCMPCFTTDHQMARKCDDCCGGKGRGKCYGPQCLCR MCMPCFTTDPNMANKCRDCCGGGKKCFGPQCLCNR- MCMPCFTTD NMA KC DCCGG   K       C |
| toxin 15 | (SEQ ID NO: 23) | |
| Consensus | (SEQ ID NO: 24) | |
| Chlorotoxin | (SEQ ID NO: 1) | |
| toxin 15 | (SEQ ID NO: 25) | |
| Consensus | (SEX ID NO 26) | |
| Insectotoxin I1 (lesser Asian Scorpion) - *Mesobuthus eupeus* | | |
| Chlorotoxin | (SEQ ID NO: 1) | 1                                    38 MCMPCFTTDHQMARKCDDCCGGKGRGKCYGPQCLCR-- MCMPCFTTRPDMAQQCRACCKG--RGKCFGPQCLCGYD MCMPCFTT   MA  C  CC G  RGKCFGPQCLC 1                                  36 MCMPCFTTDHQMARKCDDCCGGKGRGKCYGPQCLCR MCMPCFTTRPDMAQQCRACCKGRGKCFGPQCLCGYD MCMPCFTT   MA  C  CC GKGK        C |
| toxin I1 | (SEQ ID NO: 27) | |
| Consensus | (SEQ ID NO: 28) | |
| Chlorotoxin | (SEQ ID NO: 1) | |
| toxin I1 | (SEQ ID NO: 29) | |
| Consensus | (SEQ ID NO: 30) | |
| Insectotoxin 15A (lesser Asian Scorpion) - *Mesobuthus eupeus* | | |
| Chlorotoxin | (SEQ ID NO: 1) | 1                                    37 MCMPCFTTDHQMARKCDDCCGGKGRGKCYGPQCLCR- MCMPCFTTDPNMAKKCRDCCGGNG--KCFGPQCLCNR MCMPCFTTD NMAKKC DCCGG G   KCFGPQCLC 1                                   36 MCMPCFTTDHQMARKCDDCCGGKGRGKCYGPQCLCR MCMPCFTTDPNMAKKCRDCCGGNGKCFGPQCLCNR- MCMPCFTTD NMAKKC DCCGG GK         C |
| toxin 15A | (SEQ ID NO: 31) | |
| Consensus | (SEQ ID NO: 32) | |
| Chlorotoxin | (SEQ ID NO: 1) | |
| toxin 15A | (SEQ ID NO: 33) | |
| Consensus | (SEQ ID NO: 34) | |
| Neurotoxin P2 (Moroccan scorpion) - *Androctonus mauretanicus* | | |
| Chlorotoxin | (SEQ ID NO: 1) | 1                                    38 MCMPCFTTDHQMARKCDDCCGGKGRGKCYGPQCLCR-- -CGPCFTTDPYTESKCATCCGG--RGKCVGPQCLCNRI C PCFTTD     KC  CCGG  RGKC GPQCLC |
| Neurotoxin | (SEQ ID NO: 35) | |
| Consensus | (SEQ ID NO: 36) | |
| Chlorotoxin | (SEQ ID NO: 1) | 1                                  36 MCMPCFTTDHQMARKCDDCCGGKGRGKCYGPQCLCR -CGPCFTTDPYTESKCATCCGGRGKCVGPQCLCNRI C PCFTTD     KC  CCGGKGK         C |
| Neurotoxin | (SEQ ID NO: 37) | |
| Consensus | (SEQ ID NO: 38) | |
| Alignment of Chlorotoxin with all above scorpion toxins | | |
| Chlorotoxin | 1                                                50 -----------------------MCMPCFTTDHQMARKCDDCCGGKGRGK -----------------------RCKPCFTTDPQMSKKCADCCGGKGKGK -----------------------RCSPCFTTDQQMTKKCYDCCGGKGKGK MKFLYGIVFIALFLTVMFATQTDGCGPCFTTDANMARKCRECCGGIG--K -----------------------MCMPCFTTDPNMANKCRDCCGGGK--K -----------------------MCMPCFTTRPDMAQQCRACCKGRG--K -----------------------MCMPCFTTDPNMAKKCRDCCGGNG--K ------------------------CGPCFTTDPYTESKCATCCGGRG--K MCMPCFTTDPNMAKKCRDCCGGKG  K | |
| Small Toxin | | |
| Toxin LQH | | |
| Chinese | | |
| toxin 15 | | |
| toxin I1 | | |
| toxin 15A | | |
| Neurotoxin | | |
| Consensus | | |
| Chlorotoxin | (SEQ ID NO: 1) | 51 61 CYGPQCLCR-- CYGPQCLC--- CYGPQCICAPY CFGPQCLCNRI CFGPQCLCNR- CFGPQCLCGYD CFGPQCLCNR- CVGPQCLCNRI CFGPQCLCNR |
| Small Toxin | (SEQ ID NO: 15) | |
| Toxin LQH | (SEQ ID NO: 17) | |
| Chinese | (SEQ ID NO: 19) | |
| toxin I5 | (SEQ ID NO: 25) | |
| toxin I1 | (SEQ ID NO: 29) | |
| toxin 15A | (SEQ ID NO: 33) | |
| Neurotoxin | (SEQ ID NO: 37) | |
| Consensus | (SEQ ID NO: 39) | |
| Alignment of Chlorotoxin with toxins not requiring gaps to align | | |
| Chlorotoxin | (SEQ ID NO: 1) | 1                                    38 MCMPCFTTDHQMARKCDDCCGGKGRGKCYGPQCLCR-- RCKPCFTTDPQMSKKCADCCGGKGKGKCYGPQCLC--- RCSFCFTTDQQMTKKCYDCCGGKGKGKCYGPQCICAPY RC PCFTTD QMSKKC DCCGGKGKGKCYGPQCLC |
| Small Toxin | (SEQ ID NO: 15) | |
| Toxin LQH | (SEQ ID NO: 17) | |
| Consensus | (SEQ ID NO: 40) | |
| Alignment of Chlorotoxin with toxins requiring gaps (gaps removed) | | |
| Chlorotoxin | 1                                                50 -----------------------MCMPCFTTDHQMARKCDDCCGGKGRGK MKFLYGIVFIALFLTVMFATQTDGCGPCFTTDANMARKCRECCGGIGKCF -----------------------MCMPCFTTDPNMANKCRDCCGGGKKCF -----------------------MCMPCFTTRPDMAQQCRACCKGRGKCF -----------------------MCMPCFTTDPNMAKKCRDCCGGNGKCF ------------------------CGPCFTTDPYTESKCATCCGGRGKCV MCMPCFTTDPNMARKCRDCCGGRGKCF | |
| CT-Neurotox | | |
| toxin 15 | | |
| toxin I1 | | |
| toxin I5A | | |
| Neurotoxin | | |
| Consensus | | |
| Chlorotoxin | (SEQ ID NO: 1) | 51 CYGPQCLCR GPQCLCNRI GPQCLCNR- GPQCLCGYD GPQCLCNR- GPQCLCNRI GPQCLCNR |
| CT-Neurotox | (SEQ ID NO: 19) | |
| toxin I5 | (SEQ ID NO: 25) | |
| toxin I1 | (SEQ ID NO: 29) | |
| toxin 15A | (SEQ ID NO: 33) | |
| Neurotoxin | (SEQ ID NO: 37) | |
| Consensus | (SEQ ID NO: 41) | |
| Chlorotoxin Peptide 8 alignment with other scorpion toxins | | |
| Pep8-Ctlx | (SEQ ID NO: 42) | 1 CGGKGRGKCY CGGKGKGKCY CGGKGKGKCY |
| Pep8-SCX1_BUTSI | (SEQ ID NO: 43) | |
| Consensus | (SEQ ID NO: 43) | |
| Pep8-Ctlx | (SEQ ID NO: 42) | 1 CGGKGRGKCY CGGKGKGKCY CGGKGKGKCY |
| Pep8-SCX8_LEIQH | (SEQ ID NO: 43) | |
| Consensus | (SEQ ID NO: 43) | |
| Pep8-Ctlx | (SEQ ID NO: 42) | 1          12 CGGRGRGKCY-- CGGIQ--KCFGP CGG GRGKCFGP |
| Pep8-AF079059_2 | (SEQ ID NO: 44) | |
| Consensus | (SEQ ID NO: 45) | |
| Pep8-Ctlx | (SEQ ID NO: 42) | 1 CGGKGRGKCY CGGIGKCFGP CGG GK |
| Pep8-AF079059_2 | (SEQ ID NO: 44) | |
| Consensus | (SEQ ID NO: 46) | |
| Pep8-Ctlx | (SEQ ID NO: 42) | 1 12 CGGKGRGKCY-- CGG-GK--KCFGP CGGKGKGKCFGP |
| Pep8-JN0361 | (SEQ ID NO: 47) | |
| Consensus | (SEQ ID NO: 48) | |
| Pep8-Ctlx | (SEQ ID NO: 42) | 1 CGGKGRGKCY CGGGKKCFGP CGG    K |
| Pep8-JN0361 | (SEQ ID NO: 47) | |
| Consensus | (SEQ ID NO: 49) | |
| Pep8-Ctlx | (SEQ ID NO: 42) | 1         12 CGGKGRGKCY-- --CKGRGKCFGP CG KGRGKCFGP |
| Pep8-SCX1_BUTEU | (SEQ ID NO: 50) | |
| Consensus | (SEQ ID NO: 51) | |
| Pep8-Ctlx | (SEX ID NO: 42) | 1 CGGKGRGKCY CKGRGKCFGP C GKGK |
| Pep8-SCX1_BUTEU | (SEQ ID NO: 50) | |
| Consensus | (SEQ ID NO: 52) | |
| Pep8-Ctlx | (SEQ ID NO: 42) | 1            12 CGGKGRGKCY-- CGGNG--KCFGP CGG GRGKCFGP |
| Pep8-SCX5_BCTEU | (SEQ ID NO: 53) | |
| Consensus | (SEQ ID NO: 54) | |
| Pep8-Ctlx | (SEQ ID NO: 42) | 1 CGGKGRGKCY CGGNGKCFGP CGG GK |
| Pep8-SCX5_BUTEU | (SEQ ID NO: 53) | |
| Consensus | (SEQ ID NO: 55) | |
| Pep8-Ctlx | (SEQ ID NO: 42) | 1            12 CGGKGRGKCY-- CGG--RGKCVGP CGGKGRGKC GP |
| Pep8-SCXP_ANDMA | (SEQ ID NO: 56) | |
| Consensus | (SEQ ID NO: 57) | |
| Pep8-Ctlx | (SEQ ID NO: 42) | 1. CGGKGRGKCY CGGRGKCVGP CGGKGK |
| Pep8-SCXP_ANDMA | (SEQ ID NO: 56) | |
| Consensus | (SEQ ID NO: 58) | |
| | | 1                    12 |
| Pep8-Ctlx | (SEQ ID NO: 42) | CGGKGRGKCY-- CGGKGKGKCY-- CGGKGKGKCY-- CGG--IGKCFGP CGG--GKKCFGP CKG--RGKCFGP CGG--NGKCFGP CGG--RGKCVGP CGG  RGKCFGP |
| Pep8-SCX1_BUTSI | (SEQ ID NO: 43) | |
| Pep8-SCX8_LEIQH | (SEQ ID NO: 43) | |
| Pep8-AF079059_2 | (SEQ ID NO: 44) | |
| Pep8-JN0361 | (SEQ ID NO: 47) | |
| Pep8-SCX1_BUTEU | (SEQ ID NO: 50) | |
| P8p8-SCX5_BUTEU | (SEQ ID NO: 53) | |
| Pep8-SCXP_ANDMA | (SEQ ID NO: 56) | |
| Consensus | (SEQ ID NO: 59) | |
| Pep8-Ctlx | (SEQ ID NO: 42) | 1 CGGKGRGKCY CGGKGKGKCY CGGKGKGKCY CGGIGKCFGP CGGGKKCFGP CKGRGKCFGP CGGNGKCFGP CGGRGKCVGP CGGKGKCFGP |
| Pep8-SCX1_BUTSI | (SEQ ID NO: 43) | |
| Fep8-SCX8_LEIQH | (SEQ ID NO: 43) | |
| Pep8-AF079059_2 | (SEQ ID NO: 44) | |
| Pep8-JN0361 | (SEQ ID NO: 47) | |
| Pep8-SCX1_BUTEU | (SEQ ID NO: 50) | |
| Pep8-SCK5_BUTEU | (SEQ ID NO: 53) | |
| Pep8-SCXP_AKDMA | (SEQ ID NO: 56) | |
| Consensus | (SEQ ID NO: 60) | |
| Chlorotoxin Peptide 21 alignment with other scorpion toxins | | |
| Pep21-cltx | (SEQ ID NO: 61) | 1 TTDKQMAKKC TTDPQMSKKC TTD QMAKKC |
| Pep21-SCX1_BUTSI | (SEQ ID NO: 62) | |
| Consensus | (SEQ ID NO: 63) | |
| Pep21-cltx | (SEQ ID NO: 61) | 1 TTDHQMAKKC TTDQQMTKKC TTD QM KKC |
| Pep21-SCX8_LEIQH | (SEQ ID NO: 64) | |
| Consensus | (SEQ ID NO: 65) | |
| Pep21-cltx | (SEQ ID NO: 61) | 1 TTDHQMARKC TTDANMARKC TTD NMARKC |
| Pep21-AF079059_2 | (SEQ ID NO: 66) | |
| Consensus | (SEQ ID NO: 67) | |
| Pep21-clt | 1 (SEQ ID NO: 61) | TTDHQMARKC TTDPNMANKC TTD NMA KC |
| Pep21-JN0361 | (SEQ ID NO: 68) | |
| Consensus | (SEQ ID NO: 69) | |
| Pep21-cltx | (SEQ ID NO: 61) | 1 TTDHQMARKC TTRPDMAQQC TT   MA  C |
| Pep21-SCX1_BUTEU | (SEQ ID NO: 70) | |
| Consensus | (SEQ ID NO: 71) | |
| Pep21-cltx | 1 (SEQ ID NO: 61) | TTDHQMARKC TTDPNMAKKC TTD NMAKKC |
| Pep21-SCX5_BUTEU | (SEQ ID NO: 72) | |
| Consensus | (SEQ ID NO: 73) | |
| Pep21-cltx | (SEQ ID NO: 61) | 1 TTDHQMARKC TTDPYTESKC TTD     KC |
| Pep21-SCXP_ANDMA | (SEQ ID NO: 74) | |
| Consensus | (SEQ ID NO: 75) | |
| Pep21-cltx | (SEQ ID NO: 61) | 1 TTDHQMARKC TTDPQMSKKC TTDQQMTKKC TTDANMARKC TTDPNMANKC TTRPDMAQQC TTDPNMAKKC TTDPYTESKC TTDPNMAKKC |
| Pep21-SCX1_BUTSI | (SEQ ID NO: 62) | |
| Pep21-SCX3_LEIQH | (SEQ ID NO: 64) | |
| Pep21-AF079059_2 | (SEQ ID NO: 66) | |
| Pep20-JN0361 | (SEQ ID NO: 68) | |
| Pep21-SCX1_BUTEU | (SEQ ID NO: 70) | |
| Pep21-SCX5_BUTEU | (SEQ ID NO: 72) | |
| Pep21-SCXP_ANDMA | (SEQ ID NO: 74) | |
| Consensus | (SEQ ID NO: 76) | |

As used herein, the term "related scorpion toxin" refers to any of the toxins or related peptides, such as those disclosed in Table 1, displaying amino acid and/or nucleotide sequence identity to chlorotoxin. Examples of rented scorpion toxins include, but are not limited to, CT neurotoxin from *Mesobuthus martensii* (GenBank Accession AAD47373), Neurotoxin BmK 41-2 from *Buthus martensii karsch* (GenBank Accession A59356), Neurotoxin Bm12-b from *Buthus martensii* (GenBank Accession AAK16444), probable Toxin LQH 8/6 from *Leiurus quinquestriatus hebraeu* (GenBank Accession P55966), Small toxin from *Mesobuthus tamulus sindicus* (GenBank Accession P15229), the sequences of which are all herein incorporated by reference in their entirety.

Homology or sequence identity at the nucleotide or amino acid sequence level is determined by **BLAST** (**B**asic **L**ocal **A**lignment **S**earch **T**ool) analysis using the algorithm employed by the programs **blastp, blastn, blastx, tblastn** and **tblastx** (Altschul et al. (1997) Nucleic Acids Res. 25,3389-3402 and Karlin et al. (1990) Proc, Natl Acad. Sci. USA 87, 2264-2268, both fully incorporated by reference) which are tailored for sequence similarity searching. The approach used by the **BLAST** program is to first consider similar segments, with gaps (non-contiguons) and without gaps (contiguous), between a query sequence and a database sequence, then to evaluate the statistical significance of all matches that are identified and finally to summarize only those matches which satisfy a preselected threshold of significance. For a discussion of basic issues in similarity searching of sequence databases, see Altschul et al. (1994) Nature Genetics 6, 119-129 which is fully incorporated by reference. The search parameters for histogram, descriptions, alignments, expect (*i.e.,* the statistical significance threshold for reporting matches against database sequences), cutoff matrix and filter (low complexity) are at the default settings. The default scoring matrix used by blastp, blastx, tblastn, and tblastx is the **BLOSUM62** matrix (Henikoff et al. (1992) Proc. Natl. Acad. Sci. USA 89, 10915-10919, fully incorporated by reference), recommended for query sequences over eighty-five nucleotides or amino acids in length.

For **blastn**, the scoring matrix is set by the ratios of **M** (*i.e.,* the reward score for a pair of matching residues) to **N** (*i.e.*, the penalty score for mismatching residues), wherein the default values for **M** and **N** are +5 and -4, respectively. Four **blastn** parameters were adjusted as follows: Q=10 (gap creation penalty); R=10 (gap extension penalty); wink=1 (generates word hits at every wink^{th} position along the query); and gapw=16 (sets the window width within which gapped alignments are generated). The equivalent **Blastp** parameter settings were Q=9; R=2; wink=1; and gapw=32. A **Bestfit** comparison between sequences, available in the GCG package version 10.0, uses DNA parameters GAP=50 (gap creation penalty) and LEN=3 (gap extension penalty) and the equivalent settings in protein comparisons are GAP=8 and LEN=2.

The present invention encompasses the allelic variants, conservative substitution variants, and the members of the scorpion toxin peptide family, having an amino acid sequence of at least about seventy-five percent, at least about eiglity-five percent, at least about ninety percent sequence, at least about ninety-five percent, or at least about ninety-nine percent sequence identity with the entire chlorotoxin sequence set forth in SEQ ID NO: 1. Identity or homology with respect to such sequences is defined herein as the percentage of amino acid residues in the candidate sequence that are identical with the known peptides, after alignment the sequences.

Fusion proteins, or N-terminal, C-terminal or internal extensions, deletions, or insertions into the peptide sequence shall not be construed as affecting homology. Examples of such extensions include, but are not limited to, the following sequences:
HHHHHHMCMPCFTTDHQMARKCDDCCGGKGRGKCYGPQCLCR SEQ ID NO: 2),
YMCMPCFTTDHQMARKCDDCCGGKGRGKCYGPQCLCR (SEQ ID NO: 3),
YSYMCMPCFTTDHQMARKCDDCCGGKGRGKCYGPQCLCR (SEQ ID NO: 4).

The chlorotoxin peptide variants include peptides having a fragment of the amino acid sequence set forth in SEQ ID NO: 1, having at least about 7, 8, 9, 10, 15, 20, 25, 30, or 35 contiguous amino acid residues. The peptide variants further include those fragments associated with the activity of chlorotoxin. Such fragments, also referred to as polypeptides, may contain functional regions of the chlorotoxin peptide identified as regions of the amino acid sequence which correspond to known peptide domains, as well as regions of pronounced hydrophilicity. Variants may also include peptide with at least two core sequences linked to one another, in any order, with intervening amino acids removed or replaced by a linker sequence. The regions are all easily identifiable by using commonly available protein sequence analysis software such as Mac Vector (Oxford Molecular).

Contemplated peptide variants further include those containing predetermined mutations by, *e.g.*, homologous recombination, site-directed or PCR mutagenesis, and the alleles or other naturally occurring variants of the family of peptides; and derivatives wherein the peptide has been covalently modified by substitution, chemical, enzymatic or other appropriate means with a moiety other than a naturally occurring amino acid (for example a detectable moiety such as an enzyme or radioisotope). Examples of chlorotoxin variant peptides include, but are not limited to the following sequences:
MCMPCFTTDHQMARKCDDCCGGKGRGKCFGPQCLCR (SEQ ID NO: 5),
RCKFCFTTDPQMSKKCADCCGGKGKGKCYGPQCLC (SEQ ID NO: 6),
RCSPCFTTDQQMTKKCYDCCGGKGKGKCYGPQCICAPY (SEQ ID NO: 7).

### Peptide Mimetics

In another class of chlorotoxin derivatives, the present invention includes peptide mimetics that mimic the three-dimensional structure of chlorotoxin. Such peptide mimetics may have significant advantages over naturally occurring peptides including, for example, more economical production, greater chemical stability, enhanced pharmacological properties (half-life, absorption, potency, efficacy, etc.), altered specificity (*e.g.,* a broad-spectrum of biological activities), reduced antigenicity and others.

In one form, mimetics are peptide-containing molecules that mimic elements of chlorotoxin peptide secondary structure. The underlying rationale behind the use of peptide mimetics is that the peptide backbone of proteins exists chiefly to orient amino acid side chains in such a way as to facilitate molecular interactions, such as those of antibody and antigen. A peptide mimetic is expected to permit molecular interactions similar to the natural molecule. In another form, peptide analogs are commonly used in the pharmaceutical industry as non-peptide drugs with properties analogous to those of the template peptide. These types of non-peptide compounds are also referred to as peptide mimetics or peptidomimetics (Fauchere (1986) Adv. Drug Res. 15, 29-69; Veber & Freidinger (1985) Trends Neurosci. 8, 392-396; Evans et al, (1987) J. Med. Chem. 30, 1229-1239 which are incorporated herein by reference) and are usually developed with the aid of computerized molecular modeling.

Peptide mimetics that are structurally similar to therapeutically useful peptides may be used to produce an equivalent therapeutic or prophylactic effect. Generally, peptide mimetics are structurally similar to a paradigm polypeptide (*i.e.*, a polypeptide that has a biochemical property or pharmacological activity), but have one or more peptide linkages optionally replaced by a linkage by methods known in the art. Labeling of peptide mimetics usually involves covalent attachment of one or more labels, directly or through a spacer (*e.g.*, an amide group), to non-interfering positions on the peptide mimetic that are predicted by quantitative structure-activity data and molecular modeling. Such non-interfering positions generally are positions that do not form direct contacts with the macromolecules to which the peptide mimetic binds to produce the therapeutic effect. Derivitization (*e.g.*, labeling) of peptide mimetics should not substantially interfere with the desired biological or pharmacological activity of the peptide mimetic.

The use of peptide mimetics can be enhanced through the use of combinatorial chemistry to create drug libraries. The design of peptide mimetics can be aided by identifying amino acid mutations that increase or decrease binding of a peptide to, for instance, a tumor cell. Approaches that can be used include the yeast two hybrid method (see Chien et al. (1991) Proc. Natl. Acad. Sci. USA 88, 9578-9582) and using the phage display method. The two hybrid method detects protein-protein interactions in yeast (Fields et al. (1989) Nature 340, 245-246). The phage display method detects the interaction between an immobilized protein and a protein that is expressed on the surface of phages such as lambda and M13 (Amberg et al. (1993) Strategies 6, 2-4; Hogrefe et al. (1993) Gene 128, 119-126). These methods allow positive and negative selection for peptide-protein interactions and the identification of the sequences that determine these interactions.

### Pharmaceutical Compositions

Pharmaceutical compositions of the present invention can be administered via parenteral, subcutaneous, intravenous, intramuscular, intraperitoneal, transdermal or buccal routes. For example, an agent may be administered locally to a tumor via microinfusion. Alternatively, or concurrently, administration may be by the oral route. For example, chlorotoxin or a derivative thereof could be administered locally to the site of a tumor, followed by oral administration of at least one chemotherapeutic agent. The prior administration of chlorotoxin may have the effect of reducing the amount of chemotherapeutic agent necessary for successful treatment thus reducing the severe side effects associated with chemotherapeutic agents. The dosage administered will be dependent upon the age, health, and weight of the recipient, kind of concurrent treatment, if any, frequency of treatment, and the nature of the effect desired.

The present invention further includes compositions containing chlorotoxin or derivatives thereof and one or more chemotherapeutic agents that are useful in the treatment of cancer. While individual needs vary, determination of optimal ranges of effective amounts of each component is within the skill of the art. Typical dosages comprise 1.0 pg/kg body weight to 100 mg/kg body weight. The preferred dosages for systemic administration comprise 100.0 ng/kg body weight to 10.0 mg/kg body weight. The preferred dosages for direct administration to a site via microinfusion comprise 1 ng/kg body weight to 1 mg/kg body weight.

In addition to chlorotoxin and chemotherapeutic agents, the compositions of the present invention may contain suitable pharmaceutically acceptable carriers comprising excipients and auxiliaries that facilitate processing of the active compounds into preparations which can be used pharmaceutically for delivery to the site of action. Suitable formulations for parenteral administration include aqueous solutions of the active compounds in water-soluble form, for example, water-soluble salts. In addition, suspensions of the active compounds as appropriate oily injection suspensions may be administered. Suitable lipophilic solvents or vehicles include fatty oils, for example, sesame oil or synthetic fatty acid esters, for example, ethyl oleate or triglycerides. Aqueous injection suspensions may contain substances which increase the viscosity of the suspension include, for example, sodium carboxymethyl cellulose, sorbitol and dextran. Optionally, the suspension may also contain stabilizers. Liposomes can also be used to encapsulate the agent for delivery into the cell.

The pharmaceutical formulation for systemic administration according to the invention may be formulated for enteral, parenteral or topical administration. Indeed, all three types of formulations may be used simultaneously to achieve systemic administration of the active ingredient.

As mentioned above, topical administration may be used. Any common topical formulation such as a solution, suspension, gel, ointment or salve and the like may be employed. Preparation of such topical formulations are described in the art of pharmaceutical formulations as exemplified, for example, by Gennaro et al. (1995) Remington's Pharmaceutical Sciences, Mack Publishing. For topical application, the compositions could also be administered as a powder or spray, particularly in aerosol form. In a some embodiments, the compositions of this invention may be administered by inhalation. For inhalation therapy the active ingredients may be in a solution useful for administration by metered dose inhalers or in a form suitable for a dry powder inhaler. In another embodiment, the compositions are suitable for administration by bronchial lavage.

Suitable formulations for oral administration include hard or soft gelatin capsules, pills, tablets, including coated tablets, elixirs, suspensions, syrups or inhalations and controlled release forms thereof. In another embodiment, the pharmaceutical composition comprises chlorotoxin or derivatives thereof in combination with at least one sustained release form of chemotherapeutic agent. In such formulations, chlorotoxin or derivatives thereof will be distributed throughout the body, prior to release of the chemotherapeutic agents, allowing for binding of chlorotoxin to the cancer cells prior to binding of the chemotherapeutic agent to the cancer cells. Upon the delayed release of the chemotherapeutic agent from such formulations, and subsequent distribution to the site of the cancer cells, the effects of the chemotherapeutic agent may be enhanced by the earlier binding of chlorotoxin to the cancer cells. Such delayed release formulations may have the same effect as sequential administration of chlorotoxin followed by one or more chemotherapeutic agents.

### Labeled Chlorotoxin and Chlorotoxin Derivatives

The invention also includes isotopically-labeled chlorotoxin or derivatives thereof, that have one or more atoms are replaced by an atom having an atomic mass or mass number different from the atomic mass or mass number usually found in nature. Examples of isotopes that can be incorporated into compounds of the invention include isotopes of hydrogen, carbon, fluorine, phosphorous, iodine, copper, rhenium, indium, yttrium, technecium and lutetium (*i.e.*, ³H, ¹⁴C, ¹⁸F, ¹⁹F, ³¹P, ³²P, ³⁵S, ¹³¹I, ¹²⁵I, ¹²³I, ⁶⁴Cu, ¹⁸⁷Re, ¹¹¹In, ⁹⁰Y, ^{99m}Tc, ¹⁷⁷Lu). In some embodiments, isotopes which are metals (*e.g.*, copper, rhenium, indium, yttrium, technecium and lutectium) are non-covalently attached to the chlorotoxin or derivatives thereof by chelation. Examples of chelation included in the invention are chelation of a metal isotope to a polyHis region fused to chlorotoxin or a derivative thereof. Non-metal isotopes may be covalently attached to chlorotoxin or derivatives thereof using any means acceptable.

The invention also includes chlorotoxin or derivatives thereof labeled with a metal such as gadolinium (Gd). In some embodiments, a metal such as gadolinium is covalently attached to chlorotoxin or derivative thereof by chelation. Examples of chelation included in the invention are chelation of a metal such as gadolinium to a polyHis region fused to chlorotoxin or a derivative thereof.

Labeled chlorotoxin and derivatives thereof provided by this invention are also useful as radiotracers for positron emission tomography (PET) imaging or for single photon emission computerized tomography (SPBCT).

Agents of the present invention, pmdrugs thereof, and pharmaceutically acceptable salts of said agents or of said prodrugs which contain the aforementioned isotopes and/or other isotopes of other atoms are within the scope of this invention. Trititium and carbon-14 isotopes are particularly preferred for their ease of preparation and detectability. Further, substitution with heavier isotopes such as deuterium can afford certain therapeutic advantages resulting from greater metabolic stability, for example increased *in vivo* half-hie or reduced dosage requirements and, hence, may be preferred in some circumstances.

### Methods of Treatment Using Combination Chemotherapy with Chlorotoxin

This invention also includes methods for the treatment of abnormal cell growth in a mammal, including a human, comprising administering to said mammal an amount of chlorotoxin or derivative thereof, or a phamiaccutical composition comprising an amount of chlorotoxin or a derivative thereof, that is effective in enhancing the effect of a chemotherapeutic agent (*i.e.*, acts as an adjuvant for the chemotherapeutic agent) when administered prior to, or subsequent to, a chemotherapeutic agent. In one embodiment of this method, the abnormal cell growth is cancer, including, but not limited to, lung cancer, bone cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the Madder, cancer of the kidney or ureter, renal cell carcinoma, carcinoma of the renal pelvis, neoplasms of the central nervous system (CNS), neuroectodermal cancer, spinal axis tumors, glioma, meningioma, pituitary adenoma, or a combination of one or more of the foregoing cancers, In another embodiment of said method, said abnormal cell growth is a benign proliferative disease, including, but not limited to, psoriasis, benign prostate hyperpiasia, hypertrophy or restinosis.

This invention also includes methods for the treatment of abnormal cell growth in a mammal which comprises administering to said mammal, including a human, a pharmaceutical composition comprising amount of chlorotoxin or a chlorotoxin derivative and one or more chemotherapeutic agents, that is effective in enhancing the effects of the chemotherapeutic agent in inhibiting abnormal cell growth. This includes the abnormal growth and/or proliferation of cancer cells including benign and malignant cells of neoplastic diseases. Inhibition of abnormal cell growth can occur by a variety of mechanism including, but not limited to, cell death, apoptosis, inhibition of cell division, transcription, translation, transduction, etc.

As discussed above, chlorotoxin and derivatives thereof can be provided in combination, or in sequential combination with other chemotherapeutic agents that are useful in the treatment of abnormal cell growth (*e.g*., cancer). As used herein, two agents are said to be administered in combination when the two agents are administered simultaneously or are administered independently in a fashion such that the agents will act at the same time. For example, chlorotoxin or chlorotoxin derivatives can be used in combination with one or more chemotherapeutic agents selected from the following types of chemotherapeutic agents including, but not limited to, mitotic inhibitors, alkylating agents, anti-metabolites, intercalating antibiotics, growth factor inhibitors, cell cycle inhibitors, enzymes, topoisomerase inhibitors, biological response modifiers, anti-hormones, and anti-androgens.

Examples of alkylating agents include, but are not limited to, carmustine, lomustine, cyclophosphamide, ifosfamide, mechlorethamine and streptozotocin. Examples of antibiotics include, but are not limited to, bleomycin, dactinomycin, daunombicin, doxorubicin, idarubicin and plicamycin. Examples of anti-metabolites include, but are not limited to, cytarabine, fludarabine, 5-fluorouracil, 6-mercaptopurine, methotrexate and 6-thioguanine. Examples of mitotic inhibitors include, but are not limited to, navelbine, paclitaxel, vinblastine and vincristine. Examples of steroid hormones and anti-androgens include, but are not limited to, aminoglutethimides, estrogens, flutamide, goserelin, leuprolide, prednisone and tamoxifen.

Examples of pharmaceutical formulations of the above chemotherapeutic agents include, but are not limited to, BCNU (*i.e*., carmustine, 1,3-bis(2-chlorocthyl)-1-nitrosurea, BiCNU®), cisplatin (cis-platinum, cis-diamminedichloroplatinum, Platinol®), doxorubicin (hydroxyl daunorubicin, Adriamycin®), gemcytabine (difluorodeoxycyctidine, Gemzar®), hyrdoxyurea (hyroxycarbamiae, Hydrea®), paclitaxel (Taxol®), temozolomide (TMZ, Temodar®), topotecan (Hycamtin®), fluorouracil (5-fluorouracil, 5-FU, Adrucil®), vincristine (VCR, Oncovin®) and vinblastine (Velbe® or Velban®).

In practicing the methods of this invention, chlorotoxin or derivatives hereof may be used alone or in combination with other therapeutic or diagnostic agents. In certain preferred embodiments, chlorotoxin or derivatives thereof may be co-administered along with other chemotherapeutic agents typically prescribed for various types of cancer according to generally accepted oncology medical practice. The compositions of this invention can be utilized *in vivo*, ordinarily in mammals, such as humans, sheep, horses, cattle, pigs, dogs, cats, rats and mice or *in vitro*. The invention is particularly useful in the treatment of human subjects.

### Methods of Treatment Using Chlorotoxin in Combination with Radiation

The invention includes a therapeutic method comprising administration of chlorotoxin or a derivative thereof in combination with radiation therapy for the treatment of diseases associated with abnormal cell growth, such as cancer. In particular, the therapy is designed to induce apoptosis (cell death) in cancer cells, although reducing the incidence or number of metastases, and reducing tumor size also are contemplated. Tumor cell resistance to radiotherapy agents represents a major problem in clinical oncology. Thus, in the context of the present invention, it also is contemplated that combination therapy with chlorotoxin could be used on radiation resistant tumors to improve the efficacy of the radiation therapy.

As discussed above, the invention includes a method of treating cancer comprising administering to a mammal with cancer an amount of chlorotoxin or a derivative thereof in combination with ionizing radiation, both in sufficient doses that, when combined, cancer cell death is induced. In one embodiment, the presence of the chlorotoxin reduces the amount of radiation required to treat the cancer when compared to radiation treatment alone. Chlorotoxin or derivatives thereof can be provided prior to said radiation, after said radiation or concurrent with said radiation.

Radiation that causes DNA damage has been used extensively and includes what are commonly known as gamma-rays, X-rays (*e.g*., external beam radiation generated by a linear accelerator), and the directed delivery of radioisotopes to tumor cells. It is most likely that all of these factors effect a broad range of damage on DNA, on the precursors of DNA, on the replication and repair of DNA, and on the assembly and maintenance of chromosomes. For external beam radiation treatment in combination with chlorotoxin, treatment is usually given as one treatment per day. Occasionally two treatments per day will be given, where a day has been missed, or with certain cancer therapy indications. The standard dosing ranges from about 1.8 Gy to about 2.0 Gy per day, with weekly doses ranging from about 9 Gy to about 10 Cy per week. Treatment is usually given five days per week with two days off for reeovery time from the preceding week of treatment.

### Methods of Diagnosis Using Chlorotoxin

The invention includes diagnostic methods for the determination of the presence and location of abnormal cell growth in an organ or body area of a patient In one embodiment of this method, the abnormal cell growth is cancer, including, but not limited to, lung cancer, bone cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), neuroectodermal cancer, spinal axis tumors, glioma, meningioma, pituitary adenoma, or a combination of one or more of the foregoing cancers.

The present methods comprise administration of a detectable quantity of a composition comprising a detectable amount of chlorotoxin or a derivative thereof to a patient. As used herein, the term "detectable amount" refers to the amount of labeled chlorotoxin or derivative thereof administered to a patient that is sufficient to enable detection of binding of the labeled chlorotoxin or derivative thereof to one or more abnormal cells including malignant cancer cells in a tumor. As used herein, the term "imaging effective amount" refers to the amount of the labeled chlorotoxin or derivative thereof administered to a patient that is sufficient to enable imaging of binding of the labeled chlorotoxin or derivative thereof to one or more abnormal cells including malignant cancer cells in a tumor.

The invention employs isotopically-labeled chlorotoxin or derivatives thereof which, in conjunction with non-invasive neuroimaging techniques such as magnetic resonance spectroscopy (MRS) or imaging (MRI), or gamma, imaging such as positron emission tomography (PET) or single-photon emission computed tomography (SPECT), are used to identify and quantify abnormal cells *in vivo* including malignant cells in tumors. The term "*in vivo* imaging" refers to any method which permits the detection of labeled chlorotoxin or a derivative thereof as described above. For gamma imaging, the radiation emitted from the tumor or area being examined is measured and expressed either as total binding, or as a ratio in which total binding in one tissue is normalized to (for example, divided by) the total binding in another tissue or the entire body of the same subject during the same *in vivo* imaging procedure. Total binding *in vivo* is defined as the entire signal detected in a tumor or tissue by an *in vivo* imaging technique without the need for correction by a second injection of an identical quantity of labeled compound along with a large excess of unlabeled, but otherwise chemically identical compound. As used herein, the terms "subject" or "patient" refers to a mammal, preferably a human, and most preferably a human suspected of having abnormal cells, including malignant cells in a tumor.

For purposes of *in vivo* imaging, the type of detection instrument available is a major factor in selecting a given label. For instance, radioactive isotopes are particularly suitable for *in vivo* imaging in the methods of the present invention. The type of instrument used will guide the selection of the radioisotope. For instance, the radioisotope chosen must have a type of decay detectable by a given type of instrument. Another consideration relates to the half-life of the radioisotope. The half-life should be long enough so that it is still detectable at the time of maximum uptake by the target, but short enough so that the host does not sustain deleterious radiation. The isotopically-labeled chlorotoxin or derivative thereof can be detected using gamma imaging where emitted gamma irradiation of the appropriate wavelength is detected. Methods of gamma imaging include, but are not limited to, positron emission tomography (PET) imaging or for single photon emission computerized tomography (SPECT). Preferably, for SPECT detection, the chosen radiolabel will lack a particulate emission, but will produce a large number of photons. For PET detection, the radiolabel will be a positron-emitting radioisotope which will be detected by the PET camera.

In the present invention, chlorotoxin or derivatives thereof are made which are useful for *in vivo* detection and imaging of tumors. These compounds are to be used in conjunction with non-invasive neuroimaging techniques such as magnetic resonance spectroscopy (MRS) or imaging (MRI), positron emission tomography (PET), and single-photon emission computed tomography (SPECT). In accordance with this invention, chlorotoxin or derivatives thereof may be labeled with any acceptable radioisotope described above by general organic chemistry techniques known to the art (see March (1992) Advanced Organic Chemistry: Reactions, Mechanisms & Structure, Wiley). Chlorotoxin and derivatives thereof also may be radiolabeled with isotopes of copper, fluorine, carbon, bromine, etc. for PET by techniques well known in the art and are described (see Phelps (1986) Positron Emission Tomography and Autoradiography, Raven Press pages 391-450). Chlorotoxin and derivatives thereof also may be radiolabeled with acceptable isotopes such as iodine for SPECT by any of several techniques known to the art (see Kulkarni (1991) Int. J. Rad. Appl. Inst. 18, 647-648).

For example, chlorotoxin and derivatives thereof may be labeled with any suitable radioactive iodine isotope, such as, but not limited to ¹³¹I or ¹²³I by iodination of a diazotized amino derivative directly via diazonium iodide (see Greenbaum (1936) Am. J. Pharm. 108, 17-18), or by conversion of the unstable diazotized amino to the stable triazene, or by conversion of a non-radioactive halogenated precursor to a stable tri-alkyl tin derivative which then can be converted to the iodo compound by several methods well known to the art (see Chumpradit et al. (1991) J. Med. Chem, 34, 877-878 and Zhuang et al. (1994) J. Med, Chem. 37, 1406-1407).

Chlorotoxin and derivatives thereof also may be radiolabeled with known metal radiolabels, such as ⁶⁴Cu or ^{99m}Tc. Modification of the substituents to introduce ligands that bind such metal ions can be effected without undue experimentation by one of ordinary skill in the radiolabeling art including covalent attachment to a polyHis region in a modified chlorotoxin peptide or derivative thereof. The metal radiolabeled chlorotoxin or derivatives thereof can then be used to detect and image tumors.

The diagnostic methods of the present invention may use isotopes detectable by nuclear magnetic resonance spectroscopy for purposes of *in vivo* imaging and spectroscopy. Elements particularly useful in magnetic resonance spectroscopy include, but are not limited to, ¹⁹F and ¹³C. Suitable radioisotopes for purposes of this invention include, beta-emitters, gamma-emitters, positron-emitters and x-ray emitters. These radioisotopes include, but are not limited to, ¹³¹I, ¹²³I, ¹⁸F, ¹¹C, ⁷⁵Br and ⁷⁶Br.

Suitable stable isotopes for use in Magnetic Resonance Imaging (MRI) or Spectroscopy (MRS), according to this invention include, but are not limited to, ¹⁹F and ¹³C. Suitable radioisotopes for *in vitro* identification and quantification of abnormal cells including tumor cells, in a tissue biopsy or post-mortem tissue include ¹²⁵I, ¹⁴C and ³H. The preferred radiolabels are ⁶⁴Cu or ¹⁸F for use in PET *in vivo* imaging, ¹²³I or ¹³¹I for use in SPECT imaging *in vivo*, ¹⁹F for MRS and MRI and ³H or ¹⁴C for *in vitro* methods. However, any conventional method for visualizing diagnostic probes can be utilized in accordance with this invention.

Generally, the dosage of the isotopically-labeled chlorotoxin and derivatives thereof will vary depending on considerations such as age, condition, sex, and extent of disease in the patient, contraindications, if any, concomitant therapies and other variables, to be adjusted by the skilled artisan. Dosage can vary from 0.001 mg/kg to 1000 mg/kg, preferably 0.1 mg/kg to 100 mg/kg. Administration to the patient may be local or systemic and accomplished intravenous, intra-arterial, intra-thecal (via the spinal fluid), intra-cranial or the like. Administration may also be intra-dermal or intra-cavitary, depending upon the body site under examiaation.

After a sufficient time has elapsed for the labeled chlorotoxin or derivative thereof to bind with the abnormal cells, for example thirty minutes to forty-eight hours, the area of the subject under investigation is examined by routine imaging techniques such as MRS/MRI, SPECT, planar scintillation imaging, PET, and emerging imaging techniques, as The exact protocol will necessarily vary depending upon factors specific to the patient, as noted above, and depending upon the body site under examination, method of administration and type of label used, the determination of specific procedures would be routine to the skilled artisan. For brain imaging, preferably, the amount (total or specific binding) of the bound isotopically-labeled chlorotoxin or derivatives thereof is measured and compared (as a ratio) with the amount of isotopically-labeled chlorotoxin or derivatives thereof bound to the cerebellum of the patient. This ratio is then compared to the same ratio in age-matched normal brain.

Without further description, it is believed that one of ordinary skill in the art can, using the preceding description and the following illustrative examples, make and utilize the compounds of the present invention and practice the claimed methods. The following working examples describe embodiments of the present invention, and are not to be construed as limiting in any way the remainder of the disclosure.

### Example 1

### Determination of chemotherapeutic agent activity in vitro

A tissue culture method was optimized to test the effects of various chemotherapeuuc agents on multiple cancer cell lines (see Table 1). Cells were plated on 96-well microtiter tissue culture plates at a density of approximately 1000-2000 cells per well, depending on the specific cell line. Cells were allowed to adhere twenty-four hours in a 37°C, humidified cell culture incubator supplied with five percent carbon dioxide. In order to achieve a dose-response curve for each drug in each cell line, cells were treated with decreasing concentrations of a specific cytotoxic compound for two to five days. Following treatment, the cytotoxic effect of the drug was quantified using the Cell Counting Kit-8 (CCK-8) (Dojindo Inc.) according to the manufacturer's instructions. In brief, following the treatment period with the cytotoxic drug, cells were incubated with CCK-8 reagent and incubated at 37°C for one to four hours, depending on the specific cell type. After incubation, plates were read on a microplate reader at a wavelength of 490 nM. The IC₅₀ of each drug was calculated from a X-Y scatter plot of the negative log concentration of drug versus mean optical density (Table 2).

| **Table 2. Cell Line Designation, Source and Tissue Origin** | | |
|---|---|---|
| **Cell Designation** | **Cell Line Source** | **Tissue Origin** |
| D54-MG | | Human glioblastoma multiforme |
| U251-MG | | Human glioblastoma multiforme |
| SKMEL28 | ATCC (HTB-72) | Human malignant melanoma |
| SKMEL31 | ATCC (HTB-73) | Human malignant melanoma |
| PC-3 | ATCC(CRL-1435) | Human prostate tumor |
| LNCaP ATCC | (CRL-1740) Human | prostate tumor |
| NCI-H187 | ATCC (CRL-5804) | Human small cell lung carcinoma |

| **Table 3. IC₅₀ of Chemotherapeutic Agents in Multiple Cell Lines** | | |
|---|---|---|
| **Cell Line** | **Drug** | **IC₅₀** |
| D54-MG | Doxorubicin | 60,0 ng/ml |
| D54-MG | Paclitaxel | 10.5 nM |
| D54-MG | Temodar | 0.12 mM |
| D54-MG | Cisplatin | 0.010 mg/ml |
| D54-MG | 5-Fluorouracil | 0.0015 mg/ml |
| U251 | Doxorubicin | 30.0 ng/ml |
| U251 | Paclitaxel | 8.0 nM |
| U251 | Temodar | 0.15 nM |
| SKMEL28 | Doxorubicin | 40.0 ng/ml |
| SKMEL28 | Temodar | 0.03mM |
| SKMEL28 | Cisplatin | 0.008 mg/ml |
| SKMEL31 | Doxorubicin | 35.0 ng/ml |
| SKMEL31 | Paclitaxel | 25 nM |
| SKMEL31 | Temodar | 0.15 mM |
| PC-3 | Hydroxyurea | 35 mM |

### Example 2

### Effect of chlorotoxin on chemotherapeutic a gent activity in vitro

For measurement of pharmacologic effect of chlorotoxin on chemotherapeutic agents, the cell culture methodology in Example 1 was employed with the following modifications, a concentration of the chemotherapeutic agent approaching the IC₅₀ but usually just below was used in each assay. Various amounts of chlorotoxin were then titrated in combination with a concentration of chemotherapeutic agent near or below its IC₅₀ and the effect of clilorotoxi-n on the cytotoxic effects of the chamotherapeutic agent measured two to three days following adminstration. Concentration of chlorotoxin employed in this assay ranged from micoromolar down to nanomolar concentrations.

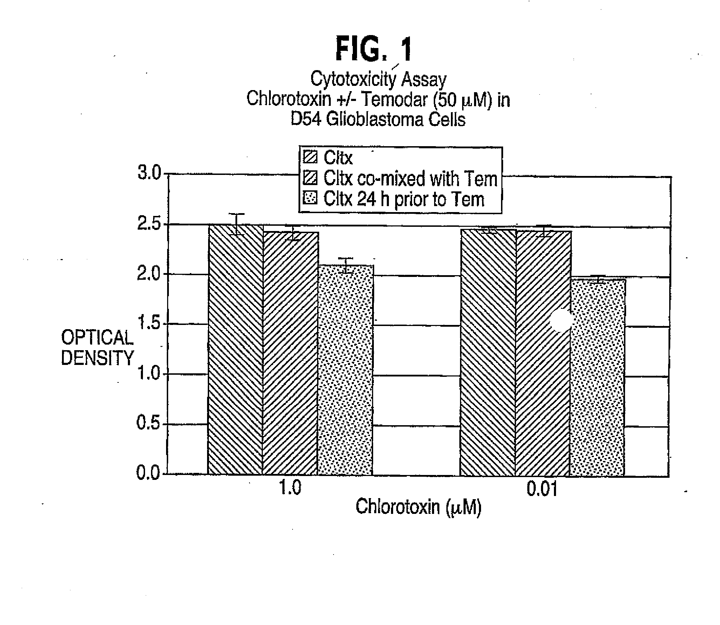

The effect of adding chlorotoxin in combination with Temodar on D54-MG cell proliferation is shown in Figure 1. The level of Temodar used in this experiment (0.050 mM) is about thirty-fold lower than the concentration necessary to kill these cell and produce a lower optical density value (see Table 2). Chloroxotoxin (TM-601) alone had no effect on cell growth. Chlorotoxin when added at the same time as Temodar did not produce any effect but when chlorotoxin was added twenty-four hours prior to Temodar, a concentration of 0.050 mM Temodar reduced cell proliferation equivalent to a level usually observed with a thirty-fold higher concentration of Temodar, These results demonstrate that administration of chlorotoxin, prior to administration of Temodar, sensitized cancer cells to the effects of Temodar.

### Example 3

### Effect of chlorotoxin on chemotherapeutic agent activity in vivo

The purpose of this study was to determine whether hydroxyurea or temodar combined with chlorotoxin were sufficient to inhibit tumor growth as indicated from *in vitro* studies with glioma cell lines. Other studies indicated that chlorotoxin, pre-incubated with human cancer cell lines, greatly sensitized the cells to temodar, a chemotherapeutic, tumor cell killing agent. Combination treatment with chlorotoxin with hydroxyurea or temodar in mice with glioma flank tumors was compared to the treatment group of hydroxyurea or temodar alone and saline alone. Hydroxyurea and temodar dosage was based on the lowest dosage (10 mg/kg body weight) used in previous studies to determine clearance from the body in the treatment of sickle cell disease paradigm in nude mice (Iyamu et al. (2001) Chemotherapy 47, 270-278).

Nude mice were ear-tagged and given an identification number were inoculated with five million U251 glioma cells in 0.10 ml mixture with five percent methyl cellulose under light anesthesia according to standard operating procedures for flank tumor inoculations (lyamu et al. (2001) Chemotherapy 47, 270-278). Flank tumors had developed and were established approximately thirty days following inoculation.

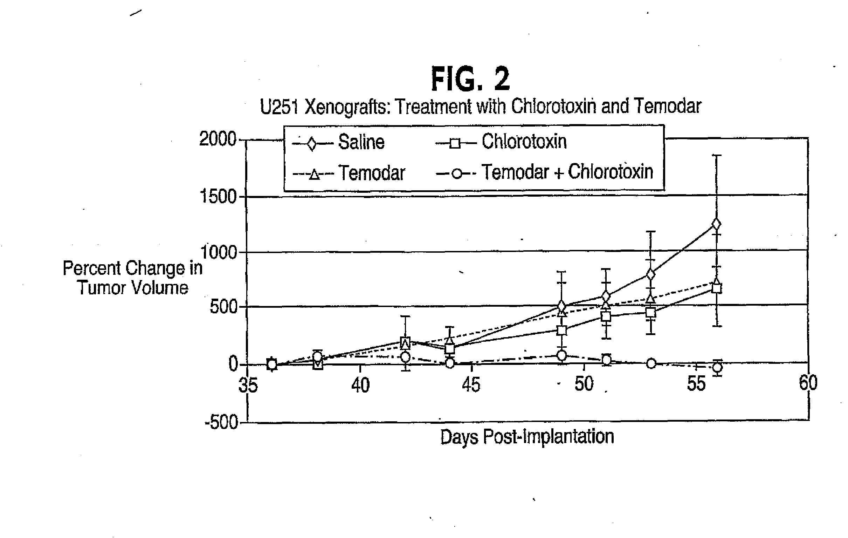

Mice with established flank tumors were each treated with 0.100 ml injection (i.p) of sterilized solutions consisting of either of saline, saline and hydroxyurea or temodar (13.2 mg/kg body weight), or saline, hydroxyurea or temodar (13.2 mg/kg) and chlorotoxin (0.080 mg/kg body weight). Tumor volume was calculated based on the measurements with the same set of calipers on the indicated days by determining the length × width × height of the tumor of non-anesthetized mice. As each animal had different-sized tumors at the beginning of the experiment, the data is presented in final form as percent change of the tumor growth from the initial date of the injection protocol. Statistical significance was determined according to a one-way ANOVA test At a level where temodar alone has little effect on the growth of the xenografted tumor, temodar combined with chlorotoxin dramatically decreased the growth of the tumor (Figure 2).

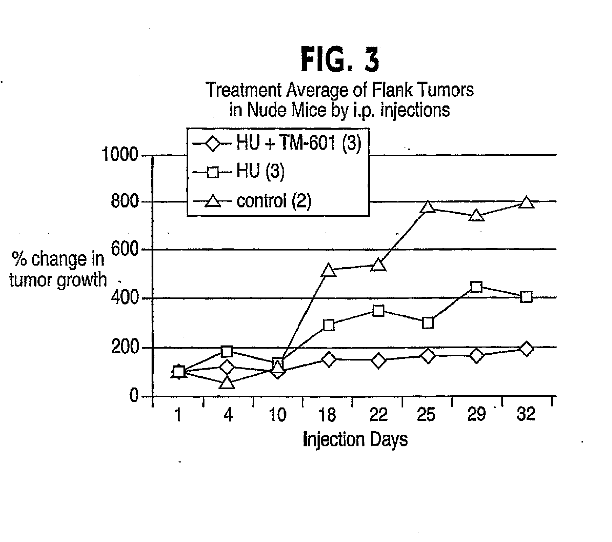

As mentioned above, the efficacy of hyrdroxyurea combined with chlorotoxin was also assessed in the same mouse flank tumor model with the exception that D54 glioma cells were used to establish the glioma flank tumor. Mice treated with chlorotoxin in combination with hydroxyurea had tumors significantly (p=0.01 at day 29 and p=0.005 at day 32) smaller in size than mice treated with either hydroxyurea alone or saline alone indicating that chlorotoxin in combination with hydroxyurea reduced tumor growth significantly more than hydroxyurea alone (Figure 3).

### Example 4

### PET imaging studies with labled chlorotoxin

The following illustrative procedure may be utilized when performing PET imaging studies on patients in the clinic. The patient is fasted for at least twelve hours allowing water intake *ad libitum* and is preinedicated with 0.3-0.4 ml Acepromazine injected mtra-muscular on the day of the experiment. A twenty-gauge, two inch intravenous catheter is inserted into the contra-lateral ulnar vein for administration of radiolabeled chlorotoxin.

The patient is positioned in the PET camera and a tracer dose of [¹⁵O]H₂O is administered via the intravenous catheter. The image thus obtained is used to insure that the patient is positioned correctly to include complete imaging of the desired areas including the tumor. Subsequently, [⁶⁴Cu] radiolabeled chlorotoxin (<20 mCi) is administered via the intravenous catheter. Following the acquisition of the total radiotracer image, an infusion is begun of the radiolabeled chlorotoxin which is evaluated at multiple dose rates (0.1, 1.0 or 10 nipk/day). After infusion for two hours, the [¹⁴Cu] radiolabeled ohlorotoxin is again injected via the catheter. Images are again acquired for up to ninety minutes. Within ten minutes of the injection of radiotracer and at the end of the imaging session, 1.0 ml blood samples are obtained for determining the plasma concentration of the radiolabeled chlorotoxin.

### Example 5

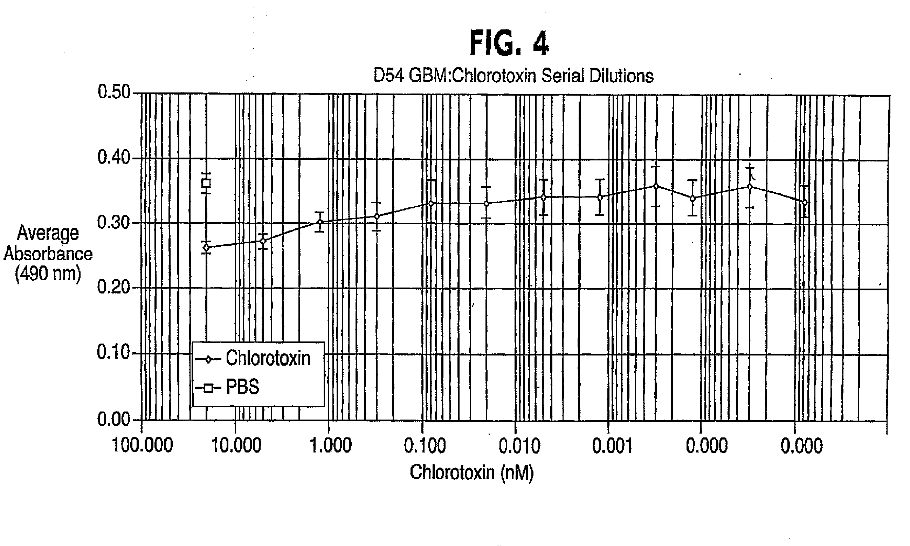

D54 glioblastoma cells were plated at a density of about 1000 cells/well in a 96-well flat bottom plate and incubated in 5% CO₂ at 37°C. After twenty-four hours chlorotoxin was added at 1:4 limiting dilutions to a final concentration of 20, 5, 1.25, 0.313, 0,078, 0.0195, 0.0049, 0.0012, 0.00031 or 0.00008 nM. Control cells received vehicle only. Twenty-four hours after treatment, the effect of chlorotoxin was quantified using the MTT mitochondrial enzyme substrate with the Cell Counting Kit-8 (CCK-8) (Dojindo Inc.) according to the manufacturer's instructions. In brief, following the treatment period with chlorotoxin, cells were incubated with CCK-8 reagent After incubation, plates were read on a microplate reader at a wavelength of 490 nm, with higher absorbance indicating greater cell viability. Figure 4 shows that chlorotoxin incubation inhibited proliferation of the D54 cells at all concentrations tested down through 0.00120 nM as evidenced by the lower number of viable cells/well versus PBS control.

### Example 6

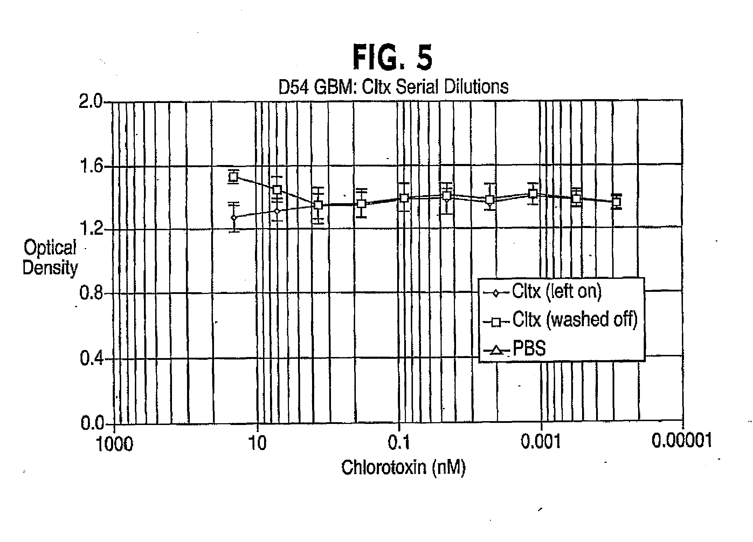

D54 glioblastoma cells were plated at a density of about 1000 cells/well in a 96-well flat bottom plate and incubated in 5% CO₂ at 37°C. After twenty-four hours chlorotoxin was added at 1:4 limiting dilutions to a final concentration (in nM) of 20, 5, 1.25, 0.313. 0.078, 0.02, 0.0049, 0.0012, 0.0003, or 0.00008. Control cells received vehicle only. After twenty-four hours, half of the cells were washed free of chlorotoxin, the medium replaced with fresh medium. Cells in both conditions, chlorotoxin left on and chlorotoxin removed, were incubated for an additional four days. Following incubation, the effect of chlorotoxin was quantified using the MTT mitochondrial enzyme substrate with the CCK-8 as in Example 1. Figure 5 shows that the long incubation time allowed me cells to overcome the effects of chlorotoxin with the additional days of proliferation and chlorotoxin did not appear to inhibit t cell proliferation in this instance.

### Example 7

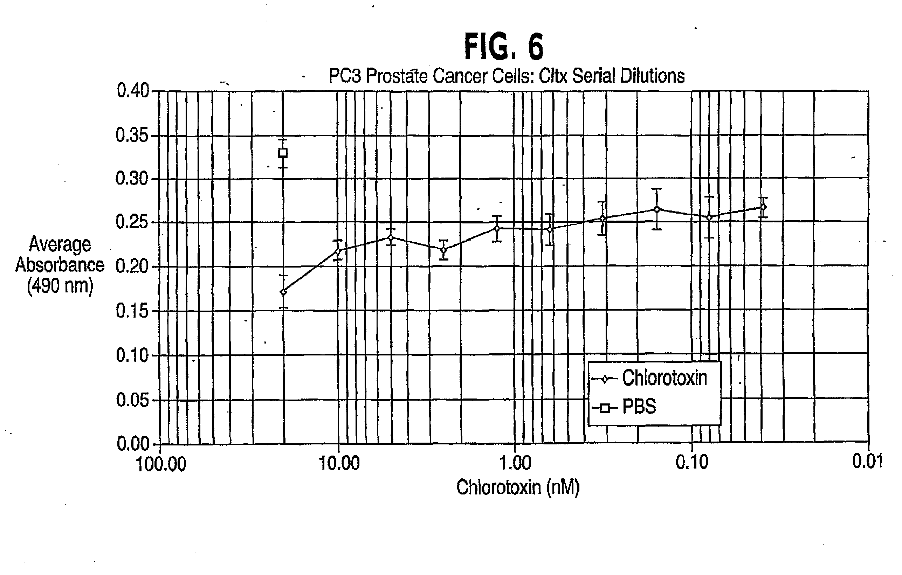

PC3 prostate cancer cells were plated at a density of about 1000 cells//well in a 96-well flat bottom plate and incubated in 5% CO₂ at 37°C. After twenty-four hours chlorotoxin was added at 1:2 limiting dilutions to a final concentration (nM) of 20, 10, 5, 2.5, 1.25, 0.625, 0.313, 0.156, 0.078, and 0.039. Control cells received PBS vehicle only. Twenty-four hours after treatment the effect of chlomtoxin was quantified using the MTT mitochondrial enzyme subxirite with the CCK-8 as in Example 1. Figure 6 shows that chlorotoxin incubation inhibited proliferation of the D54 cells at all concentrations tested as evidenced by the lower number of viable versus PUBS control

### Example 8

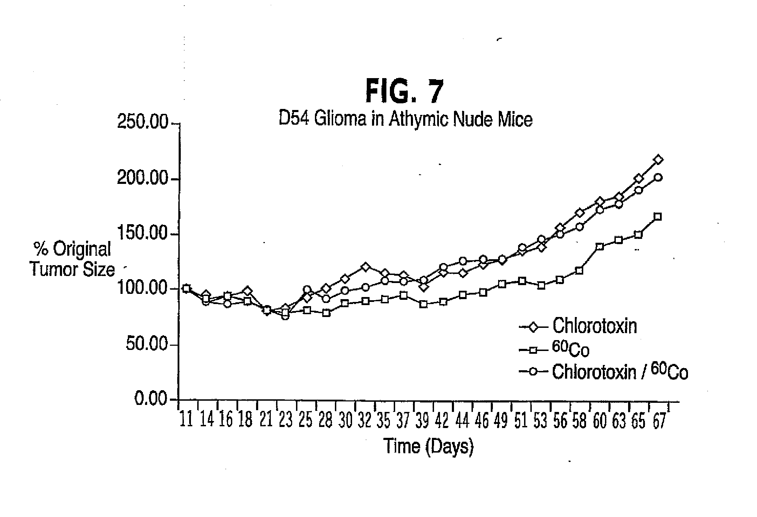

Three groups of sight athymie nude mice received a subcutaneous injection of 5 × 10⁷ human D54 glioblastoma cells in their right flank to produce human glioma flank xenografts in these mice. Animals in Groups I and III received 2.6 µg chlorotoxin (SEQ ID NO: 1) in 100 µl phosphate-buffered saline intravenously at 14, 21, 28, 35, 42 and 49 days after D54 injection. Animals in Groups II and III received 2 Gy ⁶⁰C whole-body irradiation at 15, 22, 29, 36, 43 and 50 days after D54 injection. Tumor size was measured three times weekly and is depicted in Figure 7.

### Example 9

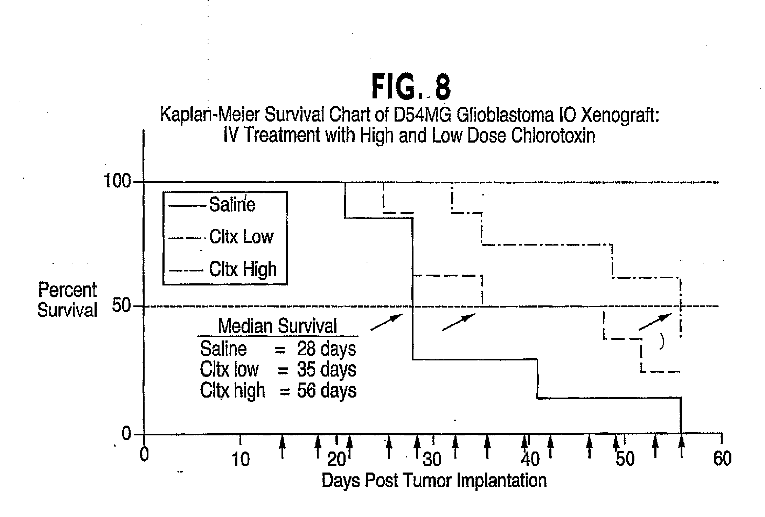

Intracranial D54MG glioma xenografts were established in athymic nude mice by the implantation of 1 × 10⁶ D54-MG cells in the brain of each subject. A treatment regimen was begun 14 days post-implantation with tail vein intravenous injections two times per week. The control group of seven mice were administered saline vehicle only. A second group of mice, comprising eight animals, were each administered a low dose of chlorotoxin of 0.2 µg/dose and a third group of mice, comprising eight animals, were administered a high dose of chlorotoxin of 2.0 µg/dose. Animals were followed until death and survival time was plotted on a Kaplan-Meier chart, indicating median survival (Figure 8). These results indicate that treatment with chlorotoxin alone substantially extends the life of a subject in an intracranial model and that this enhanced survival may be dose dependent. It is notable that administration of chlorotoxin was intravenous, demonstrating that chlorotoxincrosses the blood-brain barrier to exert its effect.

### Example 10

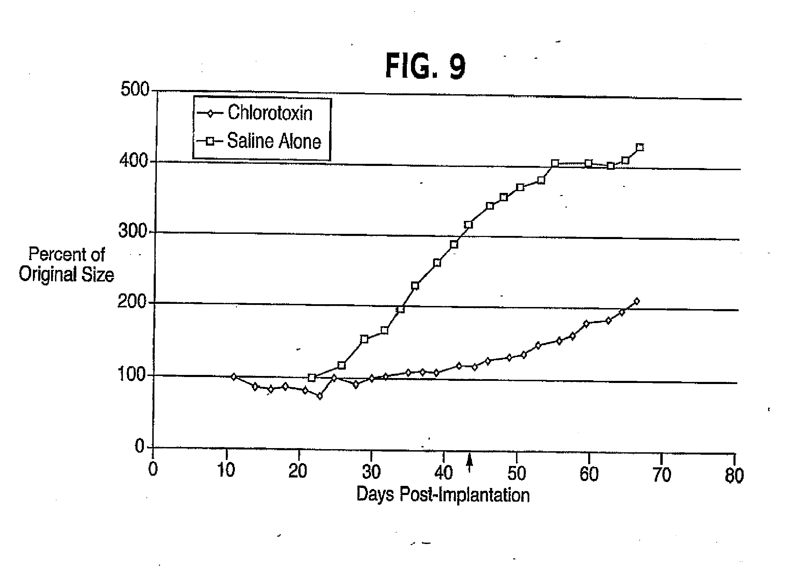

In a separate investigation, D54MG glioma xenografts were established peripherally by implanting 10 × 10⁶ D54MG cells in the flanks of athymic nude mice. Tumors were palpable at 14 days, with individual tumor volumes of approximately 43 mm³. Again, the treatment regimen was begun 14 days post-implantation with tail vein intravenous injections two times per week. The control group of seven mice were administered saline vehicle only. A second group of mice, comprising eight animals, were each administered a low dose of chlorotoxin of 0.2 µg/dose. Tumor size was measured at the time of each injection, and plotted as a percent of original tumor size (Figure 9). Intravenous treatment was ended at 42 days and the measurement of the tumors was continued for several weeks. These results demonstrate that low-dose chlorotoxin alone can dramatically decrease the tumor growth in this flank model.

### Example 11

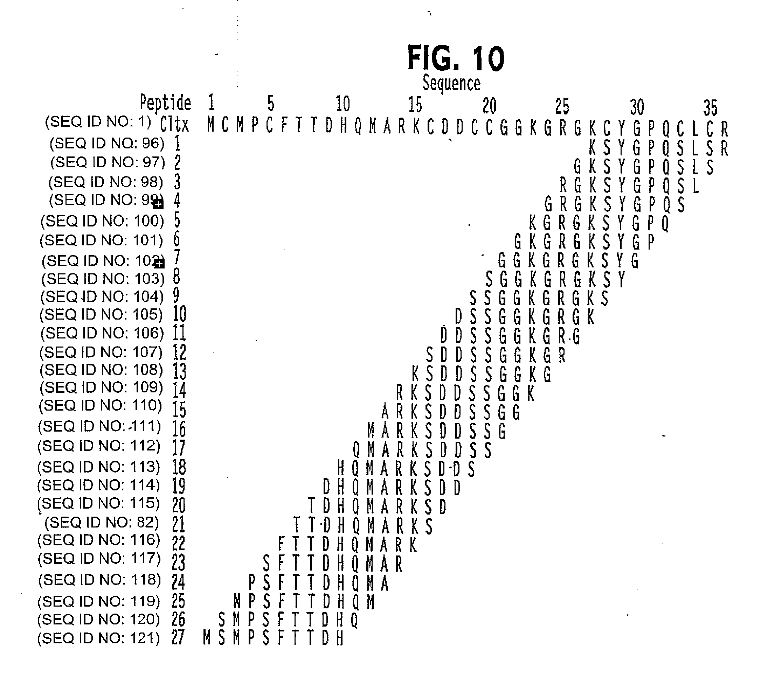

To identify core binding site sequences of chlorotoxin, twenty-seven overlapping 10-mers derived from SEQ ID NO: 1 were synthesized, starting from the C-terminus of the peptide as indicated in Figure 10. Each peptide had a biotin attached at the amino terminus to facilitate detection and each cysteine residue was replaced with a serine in order to prevent cross-linking.

Binding of the 10-mer peptides to PC3 prostate cancer cells *in vitro* was measured by incubating cultured PC3 cells with individual peptides. Binding was detected and quantified by incubating the peptide exposed cells with HRP-avidin using a commercial kit according to manufacturer's instructions.

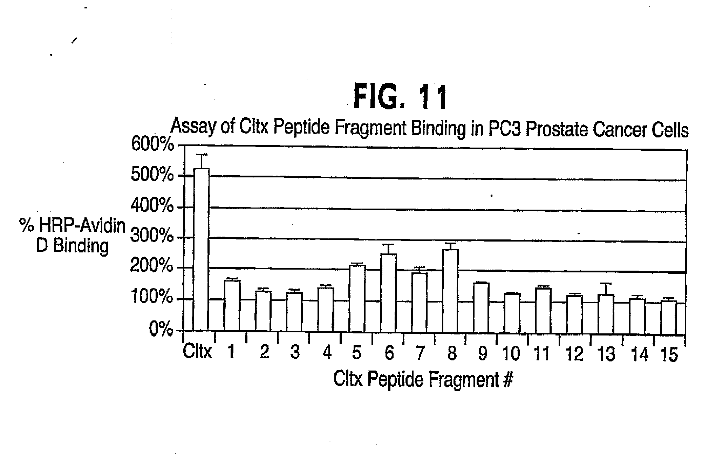

Figure 11 shows that the 10-mer peptide 4 of SEQ ID NO:1 does not bind to PC3, indicating that the lysine residue which starts peptide 5 must be the start of the binding site. Peptides 5-8 bind, but the binding is lost in peptide 9. This suggests that the tyrosine residue is another key, since this is present in peptide 8 but lost in peptide 9. This indicated that a first binding region of chlorotoxin resides within the 7-mer sequence KGRGKSY (SEQ ID NO: 8) residing at amino acid residues 23-29 of SEQ ID NO: 1 which are common to peptides 5-8.

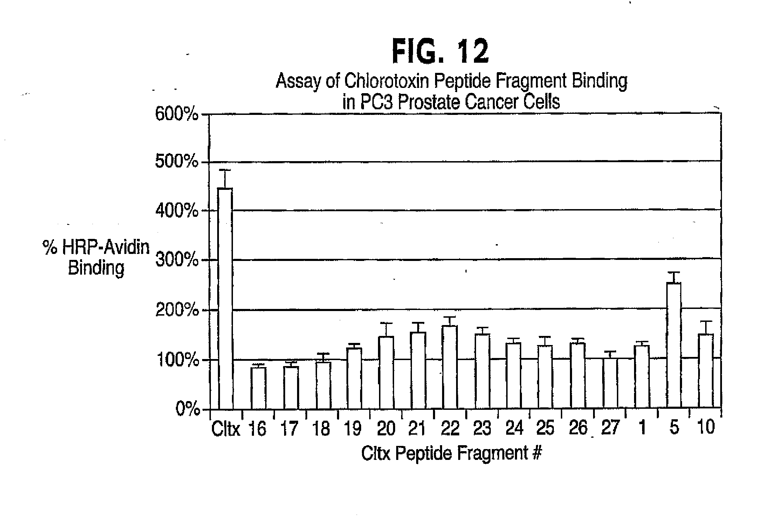

Figure 12 shows that peptide 19 of SEQ ID NO: 1 does not bind PC3 cells but peptide 20 does, indicating that the threonine residue which starts peptide 20 may be the start of a second binding site because peptides 20-24 bind most strongly. Binding decreases again in peptide 25, suggesting that the peptide 24 terminal arginine residue is another key, since this is present in peptide 24 but lost in peptide 25. This indicates that a second binding region of chlorotoxin resides within, the 9-mer sequence TDHQMAR (SEQ ID NO: 9) residing at amino acid residues 8-14 of SEQ ID NO: 1 which are common to peptides 20-24. The binding in this second core sequence is broader, which may be a reflection of very similar amino acids present at the ends of the region. For example, there are two threonine residues at peptides 20 and 21, and there is a lysine at the end of peptide 22 next to the arginine residue.

### Example 12

To determine the *in vivo* activity of these identified binding regions, 10-mer peptides 5 (amino acid residues 23-32), 12 (amino acid residues 16-25; as a negative control) and 21 (amino acid residues 7-16) of SEQ ID NO: 1 were used in a crayfish paralysis assay, an assay which is commonly used to determine the bio-activity of chlorotoxin (see DeBin et al. (1993) Am. J. Physiol. 264, C361-369). Peptides 5 and 12 failed to paralyze crayfish, while peptide 21 was effective, indicating that the site which is responsible for the paralytic effect of chlorotoxin is the region defined by peptide 21.

Additionally, several of the chlorotoxin derivatives were each analyzed in the crayfish assay and compared to chlorotoxin (Table 4). Each of these derivatives comprises the putative end-amino acids, the T and the R within the sequence corresponding to peptide 21.

| **Table 4** | | | | |
|---|---|---|---|---|
| **Peptide** | **SEQ ID** | **Crayfish Assay** | **Identity** | **Sequence Comparison** |
| Cltx | 1 | Yes | 100 % | **T**DHQMA**R** (SEQ ID NO: 9) |
| Cltx (Y/F) | 5 | Yes | 100% | **T**DHQMA**R** (SEQ ID NO: 9) |
| STP-1 | 6 | Yes | 71.4 % | **T**DPQMS**R** (SEQ ID NO: 77) |
| 6xH-Cltx | 2 | Yes | 100 % | **T**DHQMA**R** (SEQ ID NO: 9) |
| Y-Cltx | 3 | Yes | 100% | **T**DHQMA**R** (SEQ ID NO: 9) |
| YSY-Cltx | 4 | Yes | 100 % | **T**DHQMA**R** (SEQ ID NO: 9) |

### Example 13

Chlorotoxin is a 36-amino acid peptide with 8 cysteines, depicted below in bold type with the sequences of peptide number 8 (beta-region peptide) and peptide number 21 (alpha-region peptide) identified using the overlapping 10-mers in Example 12 underlined below:
MCMPCFTTDHQMARKCDDCCGGKGRCKCYGPQCLCR (SEQ ID NO: 1)

In order to confirm the identify the minimal binding sequences within the alpha and beta peptides, the entire peptides were synthesized as a 10-mer with a biotin at the amino terminus as well as shorter sequences reducing the size of the peptide by one amino acid at the amino terminus each time.

For the beta peptide, the sequences of peptide 8 noted in Table 5 were evaluated and probed for binding to U251 glioma cells:

| **Table 5** | |
|---|---|
| **Peptide** | **Sequence** |
| 8 | Biotin-GGKGRGKSYG (SEQ ID NO: 78) |
| 8a | Biotin-GKGRGKSYG (SEQ ID NO: 79) |
| 8b | Biotin-KGRGKSYG (SEQ ID NO: 80) |
| 8c | Biotin-GRGKSYG (SEQ ID NO: 81) |

For the alpha peptide, the sequences of peptide 21 noted in Table 6 were evaluated and probed for binding to U251 glioma cells:

| **Table 6** | |
|---|---|
| **Peptide** | **Sequence** |
| 21 | Biotin-TTDHQMARKS (SEQ ID NO: 82) |
| 21a | Biotin-TDHQMARKS (SEQ ID NO: 10) |
| 21b | Biotin-DHQMARKS (SEQ ID NO: 83) |
| 21c | Biotin-HQMARKS (SEQ ID NO: 84) |
| 21d | Biotin-QMARKS (SEQ ID NO: 85) |

Results demonstrated that the initial threonine residue of the alpha-region peptide is detrimental to binding but that the second threonine is crucial to binding. It was also discovered that none of the smaller peptides exhibit binding as strong as the 9-mer of peptide 21a.

### Example 14

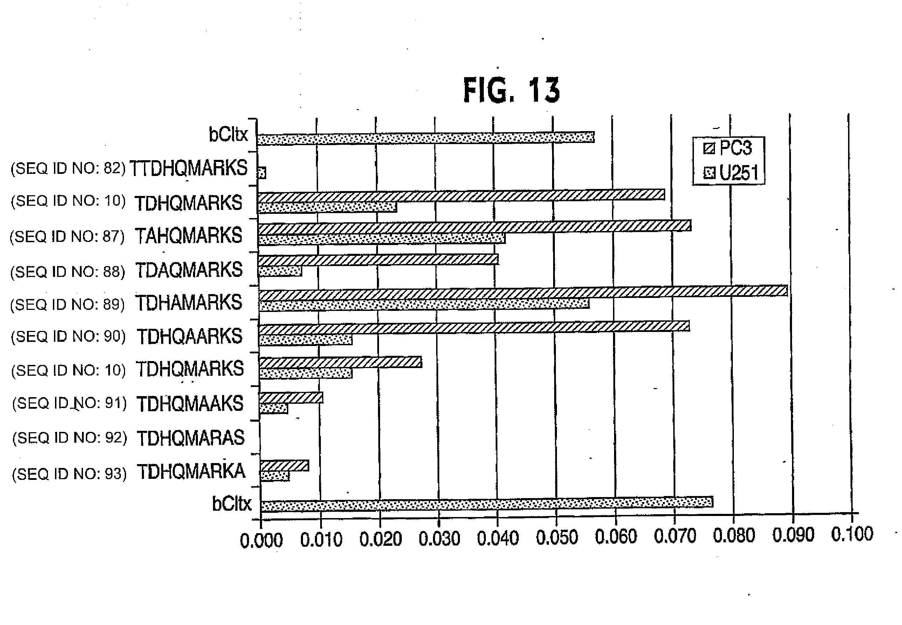

To determine the contribution of each residue to the binding properties of the alpha peptide, alanine scan variants were synthesized by replacing each amino acid of the 9-mer peptide TDHQMARKS (SEQ ID NO: 10) sequentially as depicted in Table 7. Peptide 21, the native core 9-mer, and each alanine-substituted 9-mer peptide was synthesized with a biotin at the amino terminus and evaluated for their binding versus both U251 and PC3 cells (Figure 13).

| **Table 7** | |
|---|---|
| **Peptide** | **Sequence** |
| 21 | Biotin-TTDHQMARKS (SEQ ID NO: 82) |
| 21a | Biotin-TDHQMARKS (SEQ ID NO 10) |
| 21a-A1 | Biotin-**A**DHQMARKS (SEQ ID NO: 86) |
| 21a-A2 | Biotin-T**A**HQMARKS (SEQ ID NO: 87) |
| 21a-A3 | Biotin-TD**A**QMARKS (SEQ ID NO: 88) |
| 21a-A4 | Biotin-TDH**A**MARKS (SEQ ID NO: 89) |
| 21a-A5 | Biotin-TDHQ**A**ARKS (SEQ ID NO: 90) |
| 21a-A6 | Biotin-TDHQM**A**RKS (SEQ ID NO: 10) |
| 21a-A7 | Biotin-TDHQMA**A**KS (SEQ ID NO: 91) |
| 21a-A8 | Biotin-TDHQMAR**A**S (SEQ ID NO: 92) |
| 21a-A9 | Biotin-TDHQMARK**A** (SEQ ID NO: 93) |

The pattern for U251 and PC3 binding are generally similar. Replacement of the aspartic acid (D) residue in the second position of the 9-mer increased binding of the peptide to cells and replacement of the Q residue in the fourth position produced a large increase of peptide binding to cells. Accordingly, the peptide TAHAMARKS (SEQ ID NO: 11) should be more active than the parent peptide TDHQMARKS (SEQ ID NO: 10). Based on the binding of the peptide TDHAMARKS, this binding may be equal to or greater than chlorotoxin itself.

Based upon this finding, it is expected that a variant peptide of chlorotoxín of the sequence below may be stronger in binding than the native chlorotoxin polypeptide.
MCMPCFTTAHAMARKCDDCCGGKGRCKCYGPQCLCR (SEQ ID NO: 12)

### Example 15

In order to compare binding of the short scorpion toxins, the regions homologous to peptide 21 of small toxin and probable toxin LQH-8/6 were synthesized and biotinylated for analysis in the chlorotoxin binding assay (see Table 8 for amino acid sequences of the peptides).

| **Table 8** | |
|---|---|
| **Scorpion Toxin** | **Peptide 21** |
| Chlorotoxin | TTDHQMARKS (SEQ ID NO: 82) |
| Small Toxin | TTDPQMSKK (SEQ ID NO: 94) |
| Probable Toxin LQH-8/6 | TTDQQMTKK (SEQ ID NO: 95) |

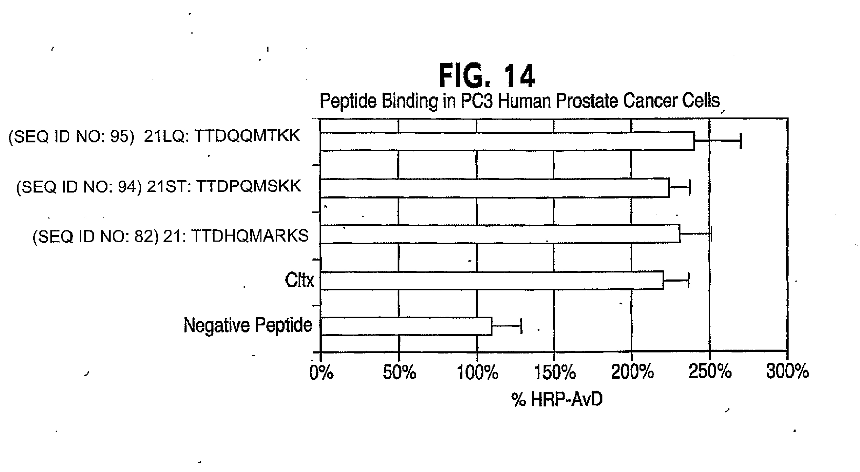

As shown in Figure 14, and in accordance with previous results, chlorotoxin exhibited significant binding in PC3 human prostate cancer cells (221.93% of background levels) and peptide 21 binding paralleled that of chlorotoxin (232.50% of background levels). Additionally, peptide 21 of small, toxin peptide (21ST) and peptide 21 of probable toxin LQH-8/6 (21LQ) demonstrated binding levels equivalent to that of full-length chlorotoxin and chlorotoxin peptide 21 (225.26% and 242.32%, respectively). Furthermore, a negative peptide containing amino acids 26-35 of chlorotoxin (SEQ ID NO: 1) exhibited binding levels comparable to background (110%). Similar results were obtained in D54 glioblastoma cells (data not shown).

The results from this study using the chlorotoxin binding assay indicate that chlorotoxin, small toxin peptide, and probable toxin LQH-8/6 bind similarly to human cancer cells *in vitro*. Table 9 below highlights amino acids conserved within the putative primary binding domain (amino acids 7-16) of the three toxin peptides.

| **Table 9** | |
|---|---|
| **Scorpion toxin** | **Amino acid sequence** |
| Chlorotoxin | **TTD**H**QM**A**RK**C (SEQ ID NO: 61) |
| Small toxin peptide | **TTD**P**QM**S**KK** (SEQ ID NO: 94) |
| Probable toxin LQH-8/6 | **TTD**Q**QM**T**KK** (SEQ ID NO: 95) |

### Example 16

The purpose of this experiment was to determine if the proliferation D54MG Glioblastoma cells, as measured by ³H-thymidine uptake, is effected by Peptide 21, a segment of the full chlorotoxin sequence. The sequence of peptide 21 and its relation to chlorotoxin is shown in the sequence below:
Chlorotoxin: MCMPCFTTDHQMARKCDDCCGGKGRGKCYGPQCLCR
Peptide 21: TTDHQMARK (SEQ ID NO: 82)
Peptide 21 (SEQ ID NO: 82) has been identified in several other reports as having binding and biological activity comparable to the full length chlorotoxin.

D54MG cells were plated in a 24 well plate at 100,000 cells/ml/well using five rows of four wells for each concentration. The cells were allowed to adhere in normal media for twenty-four hours at 37°C and 5% carbon dioxide. TM-701 was diluted to a 1 nM stock solution and added to each row at the concentrations of 0, 20, 80, 160 and 320 nM.

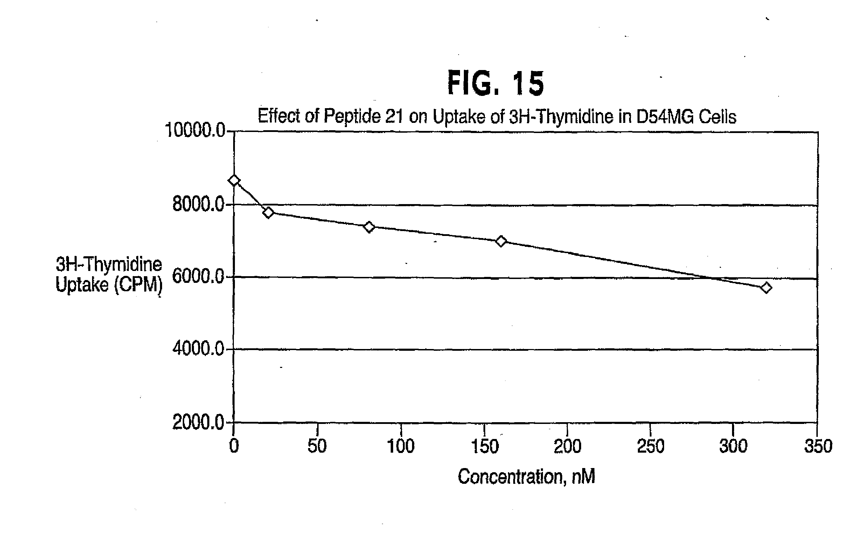

The cells and peptide 21 were allowed to incubate for 24 hours at 37°C and 5% carbon dioxide. After twenty-four hours, the cells were rinsed two times with warm PBS. Normal media was added back to the cells at 1 ml/well. One µCi of ³H-thymidine was added to each well (1 µl of 1 mCi/ml ³H-thymidine to each well). The plate was incubated for two hours at 37°C. The media and thymidine were removed and the wells were rinsed with icecold phosphate-buffered saline three times. To each well was added 1 ml of 0.3 N NaOH. The plate was incubated in the 37°C incubator for thirty minutes. Each well of 0.3 N NaOH was pipetted up and down three to four times and removed from the plate and the solution was placed in scintillation vials for counting. Scintillation fluid at four times the amount of sample was added to the vials (4 ml). Each vial was counted on the scintillation counter for one minute. The results are shown in Table 10 and Figure 15. The data demonstrates that peptide 21 behaves similar to chlorotoxin, in that the uptake of ³H-thymidine decreases in a does-dependent manner. This data also indicates that peptide 21 has an effect on the DNA synthesis in these cells.

| **Table 10** | | |
|---|---|---|
| [Peptide 21] (nM) | ³H-Thymidine uptake ± SD (CPM) | |
| 0 | 8645 ± 1218 | 1218 |
| 20 | 7795 ± 634 | 634 |
| 80 | 7412 ± 630 | 630 |
| 160 | 6983 ± 329 | 329 |
| 320 | 5782 ± 886 | 886 |

Although the present invention has been described in detail with reference to examples above, it is understood that various modifications can be made without departing from the spirit of the Accordingly, the invention is limited only by the following claims. All cited patents, patent applications and publications referred to in this application are herein incorporated by reference in their entirety.

## Claims

1. The combination of chlorotoxin or a chlorotoxin derivative and another agent for use in the treatment of abnormal cell growth in a human, wherein chlorotoxin comprises the amino acid sequence of SEQ ID NO: 1 and the chlorotoxin derivative has an amino acid sequence having at least 85% identity with SEQ ID NO: 1.

2. The combination of claim 1, wherein the amino acid sequence of the chlorotoxin derivative has at least 90% identity with SEQ ID NO: 1.

3. The combination of claim 2, wherein the amino acid sequence of the chlorotoxin derivative has at least 95% identity with SEQ ID NO: 1.

4. The combination of claim 1, wherein the amino acid sequence of the chlorotoxin derivative comprises KGRGKSY (SEQ ID NO: 8) or TTX₁X₂X₃MX₄X₅K (SEQ ID NO: 13), wherein
(a) X₁ is an acidic amino acid selected from the group consisting of aspartic acid and glutamic acid;
(b) X₂ is an amino acid selected from the group consisting of alanine, arginine, asparagine, aspartic acid, cysteine, glutamine, glutamic acid, glycine, histidine, isoleucine, leucine, lysine, proline, methionine, phenylalanine, serine, threonine, tryptophan, tyrosine, and valine;
(c) X₃ is an amide amino acid selected from the group consisting of asparagine and glutamine;
(d) X₄ is an amino acid selected from the group consisting of serine, threonine, and alanine; and
(e) X₅ is a basic amino acid selected from the group consisting of histidine, lysine and arginine.

5. The combination of any preceding claim, wherein the other agent is conjugated to the chlorotoxin or chlorotoxin derivative.

6. Use of chlorotoxin or a chlorotoxin derivative in the manufacture of a medicament for the treatment of abnormal cell growth in a human in combination with another agent, wherein chlorotoxin comprises the amino acid sequence of SEQ ID NO: 1 and the chlorotoxin derivative has an amino acid sequence having at least 85% identity with SEQ ID NO: 1.

7. Use as claimed in claim 6, modified by the features of any one of claims 12 to 5.


SEQUENCE LISTING

<110>  ALVAREZ, Vernon L.
       GRIMES, Carol A.
       GONDA, Matthew A.

<120>  Combination chemotherapy with chlorotoxin

<130>  P47897EP3/NJL

<140>
<141>

<150>  US 60/406,033
<151>  2002-08-27

<150>  US 60/384,171
<151>  2002-05-31

<160>  121

<170>  PatentIn version 3.5

<210>  1
<211>  36
<212>  PRT
<213>  Leiurus quinquestriatus


<220>
<221>  misc_feature
<223>  Chlorotoxin

<400>  1

Met Cys Met Pro Cys Phe Thr Thr Asp His Gln Met Ala Arg Lys Cys
1               5                   10                  15


Asp Asp Cys Cys Gly Gly Lys Gly Arg Gly Lys Cys Tyr Gly Pro Gln
            20                  25                  30


Cys Leu Cys Arg
        35


<210>  2
<211>  42
<212>  PRT
<213>  Leiurus quinquestriatus

<400>  2

His His His His His His Met Cys Met Pro Cys Phe Thr Thr Asp His
1               5                   10                  15


Gln Met Ala Arg Lys Cys Asp Asp Cys Cys Gly Gly Lys Gly Arg Gly
            20                  25                  30


Lys Cys Tyr Gly Pro Gln Cys Leu Cys Arg
        35                  40


<210>  3
<211>  37

<212>  PRT
<213>  Leiurus quinquestriatus

<400>  3

Tyr Met Cys Met Pro Cys Phe Thr Thr Asp His Gln Met Ala Arg Lys
1               5                   10                  15

Cys Asp Asp Cys Cys Gly Gly Lys Gly Arg Gly Lys Cys Tyr Gly Pro
            20                  25                  30

Gln Cys Leu Cys Arg
        35

<210>  4
<211>  39
<212>  PRT
<213>  Leiurus quinquestriatus

<400>  4

Tyr Ser Tyr Met Cys Met Pro Cys Phe Thr Thr Asp His Gln Met Ala
1               5                   10                  15

Arg Lys Cys Asp Asp Cys Cys Gly Gly Lys Gly Arg Gly Lys Cys Tyr
            20                  25                  30

Gly Pro Gln Cys Leu Cys Arg
        35

<210>  5
<211>  36
<212>  PRT
<213>  Artificial sequence

<220>
<223>  Synthetic - chlorotoxin variant

<400>  5

Met Cys Met Pro Cys Phe Thr Thr Asp His Gln Met Ala Arg Lys Cys
1               5                   10                  15

Asp Asp Cys Cys Gly Gly Lys Gly Arg Gly Lys Cys Phe Gly Pro Gln
            20                  25                  30

Cys Leu Cys Arg
        35

<210>  6
<211>  35
<212>  PRT
<213>  Artificial sequence

<220>
<223>  Synthetic - chlorotoxin variant

<400>  6

Arg Cys Lys Pro Cys Phe Thr Thr Asp Pro Gln Met Ser Lys Lys Cys
1               5                   10                  15

Ala Asp Cys Cys Gly Gly Lys Gly Lys Gly Lys Cys Tyr Gly Pro Gln
            20                  25                  30

Cys Leu Cys
        35

<210>  7
<211>  38
<212>  PRT
<213>  Artificial sequence

<220>
<223>  Synthetic - chlorotoxin variant

<400>  7

Arg Cys Ser Pro Cys Phe Thr Thr Asp Gln Gln Met Thr Lys Lys Cys
1               5                   10                  15

Tyr Asp Cys Cys Gly Gly Lys Gly Lys Gly Lys Cys Tyr Gly Pro Gln
            20                  25                  30

Cys Ile Cys Ala Pro Tyr
        35

<210>  8
<211>  7
<212>  PRT
<213>  Leiurus quinquestriatus

<220>
<221>  misc_feature
<223>  Derivative of Chlorotoxin:  amino acid residues 23-29

<400>  8

Lys Gly Arg Gly Lys Ser Tyr
1               5

<210>  9
<211>  7
<212>  PRT
<213>  Leiurus quinquestriatus

<220>
<221>  misc_feature
<223>  Derivative of Chlorotoxin:  amino acid residues 8-14

<400>  9

Thr Asp His Gln Met Ala Arg
1               5

<210>  10
<211>  9

<212>  PRT
<213>  Artificial sequence

<220>
<223>  Synthetic - chlorotoxin alpha peptide

<400>  10

Thr Asp His Gln Met Ala Arg Lys Ser
1               5


<210>  11
<211>  9
<212>  PRT
<213>  Artificial sequence

<220>
<223>  Synthetic - variant of chlorotoxin alpha peptide

<400>  11

Thr Ala His Ala Met Ala Arg Lys Ser
1               5


<210>  12
<211>  36
<212>  PRT
<213>  Artificial sequence

<220>
<223>  Synthetic - variant peptide of chlorotoxin

<400>  12

Met Cys Met Pro Cys Phe Thr Thr Ala His Ala Met Ala Arg Lys Cys
1               5                   10                  15


Asp Asp Cys Cys Gly Gly Lys Gly Arg Cys Lys Cys Tyr Gly Pro Gln
            20                  25                  30


Cys Leu Cys Arg
        35


<210>  13
<211>  9
<212>  PRT
<213>  Artificial

<220>
<223>  Synthetic - motif for chlorotoxin derivatives


<220>
<221>  misc_feature
<222>  (3)..(3)
<223>  Xaa can be Asp or Glu

<220>
<221>  misc_feature
<222>  (4)..(4)
<223>  Xaa can be Ala, Arg, Asn, Asp, Cys, Gln, Glu, Gly, His, Ile, Leu,
       Lys, Met, Phe, Pro, Ser, Thr, Trp, Tyr, or Val

<220>
<221>  misc_feature
<222>  (5)..(5)
<223>  Xaa can be Asn or Gln

<220>
<221>  misc_feature
<222>  (7)..(7)
<223>  Xaa can be Ala, Arg, Asn, Asp, Cys, Gln, Glu, Gly, His, Ile, Leu,
       Lys, Met, Phe, Pro, Ser, Thr, Trp, Tyr, or Val

<220>
<221>  misc_feature
<222>  (8)..(8)
<223>  Xaa can be Arg, His, or Lys

<400>  13

Thr Thr Xaa Xaa Xaa Met Xaa Xaa Lys
1               5


<210>  14
<211>  9
<212>  PRT
<213>  Leiurus quinquestriatus

<400>  14

Thr Thr Asp His Gln Met Ala Arg Lys
1               5


<210>  15
<211>  35
<212>  PRT
<213>  Mesobuthus tamulus

<400>  15

Arg Cys Lys Pro Cys Phe Thr Thr Asp Pro Gln Met Ser Lys Lys Cys
1               5                   10                  15


Ala Asp Cys Cys Gly Gly Lys Gly Lys Gly Lys Cys Tyr Gly Pro Gln
            20                  25                  30


Cys Leu Cys
        35


<210>  16
<211>  34
<212>  PRT
<213>  Artificial sequence

<220>
<223>  Small Toxin consensus sequence


<220>
<221>  MISC_FEATURE
<222>  (2)..(2)
<223>  Xaa can be Met or Lys

<220>
<221>  MISC_FEATURE

<222>  (9)..(9)
<223>  Xaa can be His or Pro

<220>
<221>  MISC_FEATURE
<222>  (16)..(16)
<223>  Xaa can be Asp or Ala

<400>  16

Cys Xaa Pro Cys Phe Thr Thr Asp Xaa Gln Met Ala Lys Lys Cys Xaa
1               5                   10                  15

Asp Cys Cys Gly Gly Lys Gly Lys Gly Lys Cys Tyr Gly Pro Gln Cys
            20                  25                  30

Leu Cys

<210>  17
<211>  38
<212>  PRT
<213>  Leiurus quinquestriatus

<400>  17

Arg Cys Ser Pro Cys Phe Thr Thr Asp Gln Gln Met Thr Lys Lys Cys
1               5                   10                  15

Tyr Asp Cys Cys Gly Gly Lys Gly Lys Gly Lys Cys Tyr Gly Pro Gln
            20                  25                  30

Cys Ile Cys Ala Pro Tyr
        35

<210>  18
<211>  34
<212>  PRT
<213>  Artificial sequence

<220>
<223>  Synthetic - Probable Toxin LQH 8/6 consensus sequence

<220>
<221>  MISC_FEATURE
<222>  (2)..(2)
<223>  Xaa can be Met or Ser

<220>
<221>  MISC_FEATURE
<222>  (9)..(9)
<223>  Xaa can be His or Gln

<220>
<221>  MISC_FEATURE
<222>  (12)..(12)
<223>  Xaa can be Ala or Thr

<220>
<221>  MISC_FEATURE
<222>  (16)..(16)

<223>  Xaa can be Asp or Tyr

<400>  18

Cys Xaa Pro Cys Phe Thr Thr Asp Xaa Gln Met Xaa Lys Lys Cys Xaa
1               5                   10                  15

Asp Cys Cys Gly Gly Lys Gly Lys Gly Lys Cys Tyr Gly Pro Gln Cys
            20                  25                  30

Ile Cys

<210>  19
<211>  61
<212>  PRT
<213>  Mesobuthus martensii

<220>
<221>  MISC_FEATURE
<222>  (1)..(61)
<223>  Xaa can be any amino acid

<400>  19

Met Lys Phe Leu Tyr Gly Ile Val Phe Ile Ala Leu Phe Leu Thr Val
1               5                   10                  15

Met Phe Ala Thr Gln Thr Asp Gly Cys Gly Pro Cys Phe Thr Thr Asp
            20                  25                  30

Ala Asn Met Ala Arg Lys Cys Arg Glu Cys Cys Gly Gly Ile Gly Xaa
        35                  40                  45

Xaa Lys Cys Phe Gly Pro Gln Cys Leu Cys Asn Arg Ile
    50                  55                  60

<210>  20
<211>  34
<212>  PRT
<213>  Artificial sequence

<220>
<223>  Synthetic - Chinese Scorpion consensus sequence

<220>
<221>  MISC_FEATURE
<222>  (2)..(2)
<223>  Xaa can be Met or Gly

<220>
<221>  MISC_FEATURE
<222>  (9)..(9)
<223>  Xaa can be His or Ala

<220>
<221>  MISC_FEATURE
<222>  (16)..(16)
<223>  Xaa can be Asp or Arg

<220>
<221>  MISC_FEATURE
<222>  (22)..(22)
<223>  Xaa can be Lys or Ile

<220>
<221>  MISC_FEATURE
<222>  (24)..(25)
<223>  Xaa can be any amino acid

<400>  20

Cys Xaa Pro Cys Phe Thr Thr Asp Xaa Asn Met Ala Arg Lys Cys Xaa
1               5                   10                  15

Asp Cys Cys Gly Gly Xaa Gly Xaa Xaa Lys Cys Phe Gly Pro Gln Cys
            20                  25                  30

Leu Cys

<210>  21
<211>  59
<212>  PRT
<213>  Mesobuthus martensii

<400>  21

Met Lys Phe Leu Tyr Gly Ile Val Phe Ile Ala Leu Phe Leu Thr Val
1               5                   10                  15

Met Phe Ala Thr Gln Thr Asp Gly Cys Gly Pro Cys Phe Thr Thr Asp
            20                  25                  30

Ala Asn Met Ala Arg Lys Cys Arg Glu Cys Cys Gly Gly Ile Gly Lys
        35                  40                  45

Cys Phe Gly Pro Gln Cys Leu Cys Asn Arg Ile
    50                  55

<210>  22
<211>  32
<212>  PRT
<213>  Artificial sequence

<220>
<223>  Chinese Scorpion consensus sequence

<220>
<221>  MISC_FEATURE
<222>  (2)..(2)
<223>  Xaa can be Met or Gly

<220>
<221>  MISC_FEATURE
<222>  (9)..(9)
<223>  Xaa can be His or Ala

<220>

<221>  MISC_FEATURE
<222>  (16)..(16)
<223>  Xaa can be Asp or Arg

<220>
<221>  MISC_FEATURE
<222>  (22)..(22)
<223>  Xaa can be Lys or Ile

<220>
<221>  MISC_FEATURE
<222>  (25)..(25)
<223>  Xaa can be Gly or Cys

<220>
<221>  MISC_FEATURE
<222>  (26)..(26)
<223>  Xaa can be Lys or Phe

<220>
<221>  MISC_FEATURE
<222>  (27)..(27)
<223>  Xaa can be Cys or Gly

<220>
<221>  MISC_FEATURE
<222>  (28)..(28)
<223>  Xaa can be Tyr or Pro

<220>
<221>  MISC_FEATURE
<222>  (29)..(29)
<223>  Xaa can be Gly or Gln

<220>
<221>  MISC_FEATURE
<222>  (30)..(30)
<223>  Xaa can be Pro or Cys

<220>
<221>  MISC_FEATURE
<222>  (31)..(31)
<223>  Xaa can be Gln or Leu

<400>  22

Cys Xaa Pro Cys Phe Thr Thr Asp Xaa Asn Met Ala Arg Lys Cys Xaa
1               5                   10                  15

Asp Cys Cys Gly Gly Xaa Gly Lys Xaa Xaa Xaa Xaa Xaa Xaa Xaa Cys
            20                  25                  30

<210>  23
<211>  37
<212>  PRT
<213>  Mesobuthus eupeus

<220>
<221>  MISC_FEATURE
<222>  (1)..(37)
<223>  Xaa can be any amino acid

<400>  23

Met Cys Met Pro Cys Phe Thr Thr Asp Pro Asn Met Ala Asn Lys Cys

1                   5                   10                  15

Arg Asp Cys Cys Gly Gly Xaa Gly Lys Xaa Lys Cys Phe Gly Pro Gln
            20                  25                  30

Cys Leu Cys Asn Arg
        35

<210>  24
<211>  35
<212>  PRT
<213>  Artificial sequence

<220>
<223>  Synthetic - Insect toxin I5 consensus sequence

<220>
<221>  MISC_FEATURE
<222>  (10)..(10)
<223>  Xaa can be His or Pro

<220>
<221>  MISC_FEATURE
<222>  (14)..(14)
<223>  Xaa can be Arg or Asn

<220>
<221>  MISC_FEATURE
<222>  (17)..(17)
<223>  Xaa can be Asp or Arg

<220>
<221>  MISC_FEATURE
<222>  (23)..(26)
<223>  Xaa can be any amino acid

<400>  24

Met Cys Met Pro Cys Phe Thr Thr Asp Xaa Asn Met Ala Xaa Lys Cys
1               5                   10                  15

Xaa Asp Cys Cys Gly Gly Xaa Gly Lys Xaa Lys Cys Phe Gly Pro Gln
            20                  25                  30

Cys Leu Cys
        35

<210>  25
<211>  35
<212>  PRT
<213>  Mesobuthus eupeus

<400>  25

Met Cys Met Pro Cys Phe Thr Thr Asp Pro Asn Met Ala Asn Lys Cys
1               5                   10                  15

Arg Asp Cys Cys Gly Gly Gly Lys Lys Cys Phe Gly Pro Gln Cys Leu
            20                  25                  30

Cys Asn Arg
        35


<210>  26
<211>  33
<212>  PRT
<213>  Artificial sequence

<220>
<223>  Synthetic - Insect toxin I5 consensus sequence


<220>
<221>  MISC_FEATURE
<222>  (10)..(10)
<223>  Xaa can be His or Pro

<220>
<221>  MISC_FEATURE
<222>  (14)..(14)
<223>  Xaa can be Arg or Asn

<220>
<221>  MISC_FEATURE
<222>  (17)..(17)
<223>  Xaa can be Asp or Arg

<220>
<221>  MISC_FEATURE
<222>  (23)..(24)
<223>  Xaa can be Lys or Gly

<220>
<221>  MISC_FEATURE
<222>  (26)..(26)
<223>  Xaa can be Gly or Cys

<220>
<221>  MISC_FEATURE
<222>  (27)..(27)
<223>  Xaa can be Lys or Phe

<220>
<221>  MISC_FEATURE
<222>  (28)..(28)
<223>  Xaa can be Cys or Gly

<220>
<221>  MISC_FEATURE
<222>  (29)..(29)
<223>  Xaa can be Tyr or Pro

<220>
<221>  MISC_FEATURE
<222>  (30)..(30)
<223>  Xaa can be Gly or Gln

<220>
<221>  MISC_FEATURE
<222>  (31)..(31)
<223>  Xaa can be Pro or Cys

<220>
<221>  MISC_FEATURE
<222>  (32)..(32)
<223>  Xaa can be Gln or Leu

<400>  26

Met Cys Met Pro Cys Phe Thr Thr Asp Xaa Asn Met Ala Xaa Lys Cys
1               5                   10                  15

Xaa Asp Cys Cys Gly Gly Xaa Xaa Lys Xaa Xaa Xaa Xaa Xaa Xaa Xaa
            20                  25                  30

Cys

<210>  27
<211>  38
<212>  PRT
<213>  Mesobuthus eupeus

<220>
<221>  MISC_FEATURE
<222>  (1)..(38)
<223>  Xaa can be any amino acid

<400>  27

Met Cys Met Pro Cys Phe Thr Thr Arg Pro Asp Met Ala Gln Gln Cys
1               5                   10                  15

Arg Ala Cys Cys Lys Gly Xaa Xaa Arg Gly Lys Cys Phe Gly Pro Gln
            20                  25                  30

Cys Leu Cys Gly Tyr Asp
        35

<210>  28
<211>  35
<212>  PRT
<213>  Artificial sequence

<220>
<223>  Synthetic - Insectotoxin I1 consensus sequence

<220>
<221>  MISC_FEATURE
<222>  (9)..(9)
<223>  Xaa can be Asp or Arg

<220>
<221>  MISC_FEATURE
<222>  (10)..(10)
<223>  Xaa can be His or Pro

<220>
<221>  MISC_FEATURE
<222>  (11)..(11)
<223>  Xaa can be Gln or Asp

<220>
<221>  MISC_FEATURE
<222>  (14)..(14)
<223>  Xaa can be Arg or Gln

<220>
<221>  MISC_FEATURE
<222>  (15)..(15)
<223>  Xaa can be Lys or Gln

<220>
<221>  MISC_FEATURE
<222>  (17)..(17)
<223>  Xaa can be Asp or Arg

<220>
<221>  MISC_FEATURE
<222>  (18)..(18)
<223>  Xaa can be Asp or Ala

<220>
<221>  MISC_FEATURE
<222>  (21)..(21)
<223>  Xaa can be Gly or Lys

<220>
<221>  MISC_FEATURE
<222>  (23)..(24)
<223>  Xaa can be any amino acid

<400>  28

Met Cys Met Pro Cys Phe Thr Thr Xaa Xaa Xaa Met Ala Xaa Xaa Cys
1               5                   10                  15

Xaa Xaa Cys Cys Xaa Gly Xaa Xaa Arg Gly Lys Cys Phe Gly Pro Gln
            20                  25                  30

Cys Leu Cys
        35

<210>  29
<211>  36
<212>  PRT
<213>  Mesobuthus eupeus

<400>  29

Met Cys Met Pro Cys Phe Thr Thr Arg Pro Asp Met Ala Gln Gln Cys
1               5                   10                  15

Arg Ala Cys Cys Lys Gly Arg Gly Lys Cys Phe Gly Pro Gln Cys Leu
            20                  25                  30

Cys Gly Tyr Asp
        35

<210>  30
<211>  33
<212>  PRT
<213>  Artificial sequence

<220>
<223>  Synthetic - Insectotoxin I1 consensus sequence

<220>
<221>  MISC_FEATURE
<222>  (9)..(9)
<223>  Xaa can be Asp or Arg

<220>
<221>  MISC_FEATURE
<222>  (10)..(10)
<223>  Xaa can be His or Pro

<220>
<221>  MISC_FEATURE
<222>  (11)..(11)
<223>  Xaa can be Gln or Asp

<220>
<221>  MISC_FEATURE
<222>  (14)..(14)
<223>  Xaa can be Arg or Gln

<220>
<221>  MISC_FEATURE
<222>  (15)..(15)
<223>  Xaa can be Lys or Gln

<220>
<221>  MISC_FEATURE
<222>  (17)..(17)
<223>  Xaa can be Asp or Arg

<220>
<221>  MISC_FEATURE
<222>  (18)..(18)
<223>  Xaa can be Asp or Arg

<220>
<221>  MISC_FEATURE
<222>  (21)..(21)
<223>  Xaa can be Gly or Lys

<220>
<221>  MISC_FEATURE
<222>  (26)..(26)
<223>  Xaa can be Gly or Cys

<220>
<221>  MISC_FEATURE
<222>  (27)..(27)
<223>  Xaa can be Lys or Phe

<220>
<221>  MISC_FEATURE
<222>  (28)..(28)
<223>  Xaa can be Cys or Gly

<220>
<221>  MISC_FEATURE
<222>  (29)..(29)
<223>  Xaa can be Tyr or Pro

<220>
<221>  MISC_FEATURE
<222>  (30)..(30)
<223>  Xaa can be Gly or Gln

<220>
<221>  MISC_FEATURE
<222>  (31)..(31)

<223>  Xaa can be Pro or Cys

<220>
<221>  MISC_FEATURE
<222>  (32)..(32)
<223>  Xaa can be Gln or Leu

<400>  30

Met Cys Met Pro Cys Phe Thr Thr Xaa Xaa Xaa Met Ala Xaa Xaa Cys
1               5                   10                  15

Xaa Xaa Cys Cys Xaa Gly Lys Gly Lys Xaa Xaa Xaa Xaa Xaa Xaa Xaa
            20                  25                  30

Cys

<210>  31
<211>  37
<212>  PRT
<213>  Mesobuthus eupeus

<220>
<221>  MISC_FEATURE
<222>  (1)..(37)
<223>  Xaa can be any amino acid

<400>  31

Met Cys Met Pro Cys Phe Thr Thr Asp Pro Asn Met Ala Lys Lys Cys
1               5                   10                  15

Arg Asp Cys Cys Gly Gly Asn Gly Xaa Xaa Lys Cys Phe Gly Pro Gln
            20                  25                  30

Cys Leu Cys Asn Arg
        35

<210>  32
<211>  35
<212>  PRT
<213>  Artificial sequence

<220>
<223>  Synthetic - Insectotoxin 15A consensus sequence

<220>
<221>  MISC_FEATURE
<222>  (10)..(10)
<223>  Xaa can be His or Pro

<220>
<221>  MISC_FEATURE
<222>  (17)..(17)
<223>  Xaa can be Asp or Arg

<220>
<221>  MISC_FEATURE
<222>  (23)..(23)

<223>  Xaa can be Lys or Asn

<220>
<221>  MISC_FEATURE
<222>  (25)..(26)
<223>  Xaa can be any amino acid

<400>  32

Met Cys Met Pro Cys Phe Thr Thr Asp Xaa Asn Met Ala Lys Lys Cys
1               5                   10                  15


Xaa Asp Cys Cys Gly Gly Xaa Gly Xaa Xaa Lys Cys Phe Gly Pro Gln
            20                  25                  30


Cys Leu Cys
        35


<210>  33
<211>  35
<212>  PRT
<213>  Mesobuthus eupeus

<400>  33

Met Cys Met Pro Cys Phe Thr Thr Asp Pro Asn Met Ala Lys Lys Cys
1               5                   10                  15


Arg Asp Cys Cys Gly Gly Asn Gly Lys Cys Phe Gly Pro Gln Cys Leu
            20                  25                  30


Cys Asn Arg
        35


<210>  34
<211>  33
<212>  PRT
<213>  Artificial sequence

<220>
<223>  Synthetic - Insectotoxin 15A consensus sequence


<220>
<221>  MISC_FEATURE
<222>  (10)..(10)
<223>  Xaa can be His or Pro

<220>
<221>  MISC_FEATURE
<222>  (17)..(17)
<223>  Xaa can be Asp or Arg

<220>
<221>  MISC_FEATURE
<222>  (23)..(23)
<223>  Xaa can be Lys or Asn

<220>
<221>  MISC_FEATURE
<222>  (26)..(26)
<223>  Xaa can be Gly or Cys

<220>
<221>  MISC_FEATURE
<222>  (27)..(27)
<223>  Xaa can be Lys or Phe

<220>
<221>  MISC_FEATURE
<222>  (28)..(28)
<223>  Xaa can be Cys or Gly

<220>
<221>  MISC_FEATURE
<222>  (29)..(29)
<223>  Xaa can be Tyr or Pro

<220>
<221>  MISC_FEATURE
<222>  (30)..(30)
<223>  Xaa can be Gly or Gln

<220>
<221>  MISC_FEATURE
<222>  (31)..(31)
<223>  Xaa can be Pro or Cys

<220>
<221>  MISC_FEATURE
<222>  (32)..(32)
<223>  Xaa can be Gln or Leu

<400>  34

Met Cys Met Pro Cys Phe Thr Thr Asp Xaa Asn Met Ala Lys Lys Cys
1               5                   10                  15

Xaa Asp Cys Cys Gly Gly Xaa Gly Lys Xaa Xaa Xaa Xaa Xaa Xaa Xaa
            20                  25                  30

Cys

<210>  35
<211>  37
<212>  PRT
<213>  Androctonus mauretanicus

<220>
<221>  MISC_FEATURE
<222>  (1)..(37)
<223>  Xaa can be any amino acid

<400>  35

Cys Gly Pro Cys Phe Thr Thr Asp Pro Tyr Thr Glu Ser Lys Cys Ala
1               5                   10                  15

Thr Cys Cys Gly Gly Xaa Xaa Arg Gly Lys Cys Val Gly Pro Gln Cys
            20                  25                  30

Leu Cys Asn Arg Ile
        35

<210>  36
<211>  34
<212>  PRT
<213>  Artificial sequence

<220>
<223>  Neurotoxin P2 consensus sequence


<220>
<221>  MISC_FEATURE
<222>  (2)..(2)
<223>  Xaa can be Met or Gly

<220>
<221>  MISC_FEATURE
<222>  (9)..(9)
<223>  Xaa can be His or Pro

<220>
<221>  MISC_FEATURE
<222>  (10)..(10)
<223>  Xaa can be Gln or Tyr

<220>
<221>  MISC_FEATURE
<222>  (11)..(11)
<223>  Xaa can be Met or Thr

<220>
<221>  MISC_FEATURE
<222>  (12)..(12)
<223>  Xaa can be Ala or Glu

<220>
<221>  MISC_FEATURE
<222>  (13)..(13)
<223>  Xaa can be Arg or Ser

<220>
<221>  MISC_FEATURE
<222>  (16)..(16)
<223>  Xaa can be Asp or Ala

<220>
<221>  MISC_FEATURE
<222>  (17)..(17)
<223>  Xaa can be Asp or Thr

<220>
<221>  MISC_FEATURE
<222>  (22)..(23)
<223>  Xaa can be any amino acid

<220>
<221>  MISC_FEATURE
<222>  (28)..(28)
<223>  Xaa can be Tyr or Val

<400>  36

Cys Xaa Pro Cys Phe Thr Thr Asp Xaa Xaa Xaa Xaa Xaa Lys Cys Xaa
1               5                   10                  15


Xaa Cys Cys Gly Gly Xaa Xaa Arg Gly Lys Cys Xaa Gly Pro Gln Cys

20                   25                   30

Leu Cys

<210>  37
<211>  35
<212>  PRT
<213>  Androctonus mauretanicus

<400>  37

Cys Gly Pro Cys Phe Thr Thr Asp Pro Tyr Thr Glu Ser Lys Cys Ala
1               5                   10                  15

Thr Cys Cys Gly Gly Arg Gly Lys Cys Val Gly Pro Gln Cys Leu Cys
            20                  25                  30

Asn Arg Ile
        35

<210>  38
<211>  32
<212>  PRT
<213>  Artificial sequence

<220>
<223>  Synthetic - Neurotoxin P2 consensus sequence

<220>
<221>  MISC_FEATURE
<222>  (2)..(2)
<223>  Xaa can be Met or Gly

<220>
<221>  MISC_FEATURE
<222>  (9)..(9)
<223>  Xaa can be His or Pro

<220>
<221>  MISC_FEATURE
<222>  (10)..(10)
<223>  Xaa can be Gln or Tyr

<220>
<221>  MISC_FEATURE
<222>  (11)..(11)
<223>  Xaa ca be Met or Thr

<220>
<221>  MISC_FEATURE
<222>  (12)..(12)
<223>  Xaa can be Ala or Glu

<220>
<221>  MISC_FEATURE
<222>  (13)..(13)
<223>  Xaa can be Arg or Ser

<220>
<221>  MISC_FEATURE
<222>  (16)..(16)

<223>  Xaa can be Asp or Ala

<220>
<221>  MISC_FEATURE
<222>  (17)..(17)
<223>  Xaa can be Asp or Thr

<220>
<221>  MISC_FEATURE
<222>  (25)..(25)
<223>  Xaa can be Gly or Cys

<220>
<221>  MISC_FEATURE
<222>  (26)..(26)
<223>  Xaa can be Lys or Val

<220>
<221>  MISC_FEATURE
<222>  (27)..(27)
<223>  Xaa can be Cys or Gly

<220>
<221>  MISC_FEATURE
<222>  (28)..(28)
<223>  Xaa can be Tyr or Pro

<220>
<221>  MISC_FEATURE
<222>  (29)..(29)
<223>  Xaa can be Gly or Gln

<220>
<221>  MISC_FEATURE
<222>  (30)..(30)
<223>  Xaa can be Pro or Cys

<220>
<221>  MISC_FEATURE
<222>  (31)..(31)
<223>  Xaa can be Gln or Leu

<400>  38

Cys Xaa Pro Cys Phe Thr Thr Asp Xaa Xaa Xaa Xaa Xaa Lys Cys Xaa
1               5                   10                  15

Xaa Cys Cys Gly Gly Lys Gly Lys Xaa Xaa Xaa Xaa Xaa Xaa Xaa Cys
            20                  25                  30

<210>  39
<211>  37
<212>  PRT
<213>  Artificial sequence

<220>
<223>  Synthetic - Toxin consensus sequence

<220>
<221>  MISC_FEATURE
<222>  (1)..(37)
<223>  Xaa can be any amino acid

<400>  39

Met Cys Met Pro Cys Phe Thr Thr Asp Pro Asn Met Ala Lys Lys Cys
1               5                   10                  15

Arg Asp Cys Cys Gly Gly Lys Gly Xaa Xaa Lys Cys Phe Gly Pro Gln
            20                  25                  30

Cys Leu Cys Asn Arg
        35

<210>  40
<211>  35
<212>  PRT
<213>  Artificial sequence

<220>
<223>  Synthetic - Toxin consensus sequence

<220>
<221>  MISC_FEATURE
<222>  (3)..(3)
<223>  Xaa can be Met, Lys or Ser

<220>
<221>  MISC_FEATURE
<222>  (10)..(10)
<223>  Xaa can be His, Pro, or Gln

<220>
<221>  MISC_FEATURE
<222>  (17)..(17)
<223>  Xaa can be Asp, Ala, or Tyr

<400>  40

Arg Cys Xaa Pro Cys Phe Thr Thr Asp Xaa Gln Met Ser Lys Lys Cys
1               5                   10                  15

Xaa Asp Cys Cys Gly Gly Lys Gly Lys Gly Lys Cys Tyr Gly Pro Gln
            20                  25                  30

Cys Leu Cys
        35

<210>  41
<211>  35
<212>  PRT
<213>  Artificial sequence

<220>
<223>  Synthetic -Toxin consensus sequence

<400>  41

Met Cys Met Pro Cys Phe Thr Thr Asp Pro Asn Met Ala Arg Lys Cys
1               5                   10                  15

Arg Asp Cys Cys Gly Gly Arg Gly Lys Cys Phe Gly Pro Gln Cys Leu
            20                  25                  30

Cys Asn Arg
        35


<210>  42
<211>  10
<212>  PRT
<213>  Artificial sequence

<220>
<223>  Synthetic - Pep8-Ctlx

<400>  42

Cys Gly Gly Lys Gly Arg Gly Lys Cys Tyr
1               5                   10


<210>  43
<211>  10
<212>  PRT
<213>  Artificial sequence

<220>
<223>  Synthetic - Pep8-SCX1_BUTSI

<400>  43

Cys Gly Gly Lys Gly Lys Gly Lys Cys Tyr
1               5                   10


<210>  44
<211>  10
<212>  PRT
<213>  Artificial sequence

<220>
<223>  Synthetic - Pep8-AF079059_2

<400>  44

Cys Gly Gly Ile Gly Lys Cys Phe Gly Pro
1               5                   10


<210>  45
<211>  12
<212>  PRT
<213>  Artificial sequence

<220>
<223>  Synthetic - Chlorotoxin Peptide 8 consensus sequence


<220>
<221>  MISC_FEATURE
<222>  (4)..(4)
<223>  Xaa can be Lys or Ile

<400>  45

Cys Gly Gly Xaa Gly Arg Gly Lys Cys Phe Gly Pro
1               5                   10


<210>  46
<211>  6

<212>  PRT
<213>  Artificial sequence

<220>
<223>  Synthetic - Chlorotoxin Peptide 8 consensus sequence


<220>
<221>  MISC_FEATURE
<222>  (4)..(4)
<223>  Xaa can be Lys or Ile

<400>  46

Cys Gly Gly Xaa Gly Lys
1               5


<210>  47
<211>  10
<212>  PRT
<213>  Artificial sequence

<220>
<223>  Synthetic - Pep8-NJ0361 sequence

<400>  47

Cys Gly Gly Gly Lys Lys Cys Phe Gly Pro
1               5                   10


<210>  48
<211>  12
<212>  PRT
<213>  Artificial sequence

<220>
<223>  Synthetic - Chlorotoxin Peptide 8 consensus sequence

<400>  48

Cys Gly Gly Lys Gly Lys Gly Lys Cys Phe Gly Pro
1               5                   10


<210>  49
<211>  6
<212>  PRT
<213>  Artificial sequence

<220>
<223>  Synthetic - Chlorotoxin Peptide 8 consensus sequence


<220>
<221>  MISC_FEATURE
<222>  (4)..(5)
<223>  Xaa can be Lys or Gly

<400>  49

Cys Gly Gly Xaa Xaa Lys
1               5


<210>  50
<211>  10

<212>  PRT
<213>  Artificial sequence

<220>
<223>  Synthetic - Pep8-SCX1_BUTEU sequence

<400>  50

Cys Lys Gly Arg Gly Lys Cys Phe Gly Pro
1               5                   10


<210>  51
<211>  12
<212>  PRT
<213>  Artificial sequence

<220>
<223>  Synthetic - chlorotoxin peptide 8 consensus sequence


<220>
<221>  MISC_FEATURE
<222>  (3)..(3)
<223>  Xaa can be Gly or Cys

<400>  51

Cys Gly Xaa Lys Gly Arg Gly Lys Cys Phe Gly Pro
1               5                   10


<210>  52
<211>  6
<212>  PRT
<213>  Artificial sequence

<220>
<223>  Synthetic - chlorotoxin peptide 8 consensus sequence


<220>
<221>  MISC_FEATURE
<222>  (2)..(2)
<223>  Xaa can be Gly or Lys

<400>  52

Cys Xaa Gly Lys Gly Lys
1               5


<210>  53
<211>  10
<212>  PRT
<213>  Artificial sequence

<220>
<223>  Synthetic - Pep8-SCX5_BUTEU sequence

<400>  53

Cys Gly Gly Asn Gly Lys Cys Phe Gly Pro
1               5                   10


<210>  54
<211>  12

<212>  PRT
<213>  Artificial sequence

<220>
<223>  Synthetic - chlorotoxin peptide 8 consensus sequence


<220>
<221>  MISC_FEATURE
<222>  (4)..(4)
<223>  Xaa can be Lys or Asn

<400>  54

Cys Gly Gly Xaa Gly Arg Gly Lys Cys Phe Gly Pro
1               5                   10


<210>  55
<211>  6
<212>  PRT
<213>  Artificial sequence

<220>
<223>  Synthetic - chlorotoxin peptide 8 consensus sequence


<220>
<221>  MISC_FEATURE
<222>  (4)..(4)
<223>  Xaa can be Lys or Asn

<400>  55

Cys Gly Gly Xaa Gly Lys
1               5


<210>  56
<211>  10
<212>  PRT
<213>  Artificial sequence

<220>
<223>  Synthetic - Pep8-SCXP_ANDMA sequence

<400>  56

Cys Gly Gly Arg Gly Lys Cys Val Gly Pro
1               5                   10


<210>  57
<211>  12
<212>  PRT
<213>  Artificial sequence

<220>
<223>  Synthetic - chlorotoxin peptide 8 consensus sequence


<220>
<221>  MISC_FEATURE
<222>  (10)..(10)
<223>  Xaa can be Tyr or Val

<400>  57

Cys Gly Gly Lys Gly Arg Gly Lys Cys Xaa Gly Pro
1               5                   10


<210>  58
<211>  6
<212>  PRT
<213>  Artificial sequence

<220>
<223>  Synthetic - chlorotoxin peptide 8 consensus sequence

<400>  58

Cys Gly Gly Lys Gly Lys
1               5


<210>  59
<211>  12
<212>  PRT
<213>  Artificial sequence

<220>
<223>  Synthetic - chlorotoxin peptide 8 consensus sequence


<220>
<221>  MISC_FEATURE
<222>  (4)..(5)
<223>  Xaa can be Lys or Gly

<400>  59

Cys Gly Gly Xaa Xaa Arg Gly Lys Cys Phe Gly Pro
1               5                   10


<210>  60
<211>  10
<212>  PRT
<213>  Artificial sequence

<220>
<223>  Synthetic - chlorotoxin peptide 8 consensus sequence

<400>  60

Cys Gly Gly Lys Gly Lys Cys Phe Gly Pro
1               5                   10


<210>  61
<211>  10
<212>  PRT
<213>  Artificial sequence

<220>
<223>  Synthetic - chlorotoxin peptide 21 sequence

<400>  61

Thr Thr Asp His Gln Met Ala Arg Lys Cys
1               5                   10


<210>  62
<211>  10

<212>  PRT
<213>  Artificial sequence

<220>
<223>  Synthetic - Pep21-SCX1-BUTSI sequence

<400>  62

Thr Thr Asp Pro Gln Met Ser Lys Lys Cys
1               5                   10


<210>  63
<211>  10
<212>  PRT
<213>  Artificial sequence

<220>
<223>  Synthetic - chlorotoxin peptide 21 consensus sequence


<220>
<221>  MISC_FEATURE
<222>  (4)..(4)
<223>  Xaa can be His or Pro

<400>  63

Thr Thr Asp Xaa Gln Met Ala Lys Lys Cys
1               5                   10


<210>  64
<211>  10
<212>  PRT
<213>  Artificial sequence

<220>
<223>  Synthetic - Pep21-SCX8_LEIQH sequence

<400>  64

Thr Thr Asp Gln Gln Met Thr Lys Lys Cys
1               5                   10


<210>  65
<211>  10
<212>  PRT
<213>  Artificial sequence

<220>
<223>  Synthetic - chlorotoxin peptide 21 consensus sequence


<220>
<221>  MISC_FEATURE
<222>  (4)..(4)
<223>  Xaa can be His or Gln

<220>
<221>  MISC_FEATURE
<222>  (7)..(7)
<223>  Xaa can be Ala or Thr

<400>  65

Thr Thr Asp Xaa Gln Met Xaa Lys Lys Cys

1                   5                   10


<210>  66
<211>  10
<212>  PRT
<213>  Artificial sequence

<220>
<223>  Synthetic - Pep21-AF079059_2 sequence

<400>  66

Thr Thr Asp Ala Asn Met Ala Arg Lys Cys
1               5                   10


<210>  67
<211>  10
<212>  PRT
<213>  Artificial sequence

<220>
<223>  Synthetic - chlorotoxin peptide 21 consensus sequence


<220>
<221>  MISC_FEATURE
<222>  (4)..(4)
<223>  Xaa can be His or Ala

<400>  67

Thr Thr Asp Xaa Asn Met Ala Arg Lys Cys
1               5                   10


<210>  68
<211>  10
<212>  PRT
<213>  Artificial sequence

<220>
<223>  Synthetic - Pep21-JN0361 sequence

<400>  68

Thr Thr Asp Pro Asn Met Ala Asn Lys Cys
1               5                   10


<210>  69
<211>  10
<212>  PRT
<213>  Artificial sequence

<220>
<223>  Synthetic - chlorotoxin peptide 21 consensus sequence


<220>
<221>  MISC_FEATURE
<222>  (4)..(4)
<223>  Xaa can be either His or Pro

<220>
<221>  MISC_FEATURE
<222>  (8)..(8)

<223>  Xaa can be Arg or Asn

<400>  69

Thr Thr Asp Xaa Asn Met Ala Xaa Lys Cys
1               5                   10


<210>  70
<211>  10
<212>  PRT
<213>  Artificial sequence

<220>
<223>  Synthetic - Pep21-SCX1_BUTEU sequence

<400>  70

Thr Thr Arg Pro Asp Met Ala Gln Gln Cys
1               5                   10


<210>  71
<211>  10
<212>  PRT
<213>  Artificial sequence

<220>
<223>  Synthetic - chlorotoxin peptide 21 consensus sequence


<220>
<221>  MISC_FEATURE
<222>  (3)..(3)
<223>  Xaa can be Asp or Arg

<220>
<221>  MISC_FEATURE
<222>  (4)..(4)
<223>  Xaa can be His or Pro

<220>
<221>  MISC_FEATURE
<222>  (5)..(5)
<223>  Xaa can be Gln or Asp

<220>
<221>  MISC_FEATURE
<222>  (8)..(8)
<223>  Xaa can be Arg or Gln

<220>
<221>  MISC_FEATURE
<222>  (9)..(9)
<223>  Xaa can be Lys or Gln

<400>  71

Thr Thr Xaa Xaa Xaa Met Ala Xaa Xaa Cys
1               5                   10


<210>  72
<211>  10
<212>  PRT
<213>  Artificial sequence

<220>

<223>  Synthetic - Pep21-SCX5_BUTEU sequence

<400>  72

Thr Thr Asp Pro Asn Met Ala Lys Lys Cys
1               5                   10


<210>  73
<211>  10
<212>  PRT
<213>  Artificial sequence

<220>
<223>  Synthetic - chlorotoxin peptide 21 consensus sequence


<220>
<221>  MISC_FEATURE
<222>  (4)..(4)
<223>  Xaa can be His or Pro

<400>  73

Thr Thr Asp Xaa Asn Met Ala Lys Lys Cys
1               5                   10


<210>  74
<211>  10
<212>  PRT
<213>  Artificial sequence

<220>
<223>  Synthetic - Pep21-SCXP_ANDMA sequence

<400>  74

Thr Thr Asp Pro Tyr Thr Glu Ser Lys Cys
1               5                   10


<210>  75
<211>  10
<212>  PRT
<213>  Artificial sequence

<220>
<223>  Synthetic - chlorotoxin peptide 21 consensus sequence


<220>
<221>  MISC_FEATURE
<222>  (4)..(4)
<223>  Xaa can be His or Pro

<220>
<221>  MISC_FEATURE
<222>  (5)..(5)
<223>  Xaa can be Gln or Tyr

<220>
<221>  MISC_FEATURE
<222>  (6)..(6)
<223>  Xaa can be Met or Thr

<220>
<221>  MISC_FEATURE

<222>  (7)..(7)
<223>  Xaa can be Ala or Glu

<220>
<221>  MISC_FEATURE
<222>  (8)..(8)
<223>  Xaa can be Arg or Ser

<400>  75

Thr Thr Asp Xaa Xaa Xaa Xaa Xaa Lys Cys
1               5                   10


<210>  76
<211>  10
<212>  PRT
<213>  Artificial sequence

<220>
<223>  Synthetic - chlorotoxin peptide 21 consensus sequence

<400>  76

Thr Thr Asp Pro Asn Met Ala Lys Lys Cys
1               5                   10


<210>  77
<211>  7
<212>  PRT
<213>  Artificial sequence

<220>
<223>  Synthetic - chlorotoxin derivative STP-1

<400>  77

Thr Asp Pro Gln Met Ser Arg
1               5


<210>  78
<211>  10
<212>  PRT
<213>  Artificial sequence

<220>
<223>  Synthetic - peptide 8 sequences

<400>  78

Gly Gly Lys Gly Arg Gly Lys Ser Tyr Gly
1               5                   10


<210>  79
<211>  9
<212>  PRT
<213>  Artificial sequence

<220>
<223>  Synthetic - peptide 8a sequence

<400>  79

Gly Lys Gly Arg Gly Lys Ser Tyr Gly
1               5

<210>  80
<211>  8
<212>  PRT
<213>  Artificial sequence

<220>
<223>  Synthetic - peptide 8b sequence

<400>  80

Lys Gly Arg Gly Lys Ser Tyr Gly
1               5


<210>  81
<211>  7
<212>  PRT
<213>  Artificial sequence

<220>
<223>  Synthetic - peptide 8c sequence

<400>  81

Gly Arg Gly Lys Ser Tyr Gly
1               5


<210>  82
<211>  10
<212>  PRT
<213>  Artificial sequence

<220>
<223>  Synthetic - peptide 21 sequence

<400>  82

Thr Thr Asp His Gln Met Ala Arg Lys Ser
1               5                   10


<210>  83
<211>  8
<212>  PRT
<213>  Artificial sequence

<220>
<223>  Synthetic - peptide 21b sequence

<400>  83

Asp His Gln Met Ala Arg Lys Ser
1               5


<210>  84
<211>  7
<212>  PRT
<213>  Artificial sequence

<220>
<223>  Synthetic - peptide 21c sequence

<400>  84

His Gln Met Ala Arg Lys Ser
1               5


<210>  85
<211>  6
<212>  PRT
<213>  Artificial sequence

<220>
<223>  Synthetic - peptide 21d sequence

<400>  85

Gln Met Ala Arg Lys Ser
1               5


<210>  86
<211>  9
<212>  PRT
<213>  Artificial sequence

<220>
<223>  Synthetic - peptide 21a-A1 sequence

<400>  86

Ala Asp His Gln Met Ala Arg Lys Ser
1               5


<210>  87
<211>  9
<212>  PRT
<213>  Artificial sequence

<220>
<223>  Synthetic - peptide 21a-A2 sequence

<400>  87

Thr Ala His Gln Met Ala Arg Lys Ser
1               5


<210>  88
<211>  9
<212>  PRT
<213>  Artificial sequence

<220>
<223>  Synthetic - peptide 21a-A3 sequence

<400>  88

Thr Asp Ala Gln Met Ala Arg Lys Ser
1               5


<210>  89
<211>  9
<212>  PRT
<213>  Artificial sequence

<220>
<223>  Synthetic - peptide 21a-A4 sequence

<400>  89

Thr Asp His Ala Met Ala Arg Lys Ser
1               5


<210>  90
<211>  9
<212>  PRT
<213>  Artificial sequence

<220>
<223>  Synthetic - peptide 21a-A5 sequence

<400>  90

Thr Asp His Gln Ala Ala Arg Lys Ser
1               5


<210>  91
<211>  9
<212>  PRT
<213>  Artificial sequence

<220>
<223>  Synthetic - peptide 21a-A7 sequence

<400>  91

Thr Asp His Gln Met Ala Ala Lys Ser
1               5


<210>  92
<211>  9
<212>  PRT
<213>  Artificial sequence

<220>
<223>  Synthetic - peptide 21a-A8 sequence

<400>  92

Thr Asp His Gln Met Ala Arg Ala Ser
1               5


<210>  93
<211>  9
<212>  PRT
<213>  Artificial sequence

<220>
<223>  Synthetic - peptide 21a-A9 sequence

<400>  93

Thr Asp His Gln Met Ala Arg Lys Ala
1               5


<210>  94
<211>  9
<212>  PRT
<213>  Mesobuthus tamulus sindicus

<220>
<221>  misc_feature
<222>  (1)..(9)
<223>  GenBank Accession No. P15229, small toxin

<400>  94

Thr Thr Asp Gln Gln Met Ser Lys Lys
1               5


<210>  95
<211>  9
<212>  PRT
<213>  Leiurus quinquestriatus hebraeu


<220>
<221>  misc_feature
<222>  (1)..(9)
<223>  GenBank Accession No. P55966, probable toxin

<400>  95

Thr Thr Asp Pro Gln Met Ser Lys Lys
1               5


<210>  96
<211>  10
<212>  PRT
<213>  Artificial Sequence

<220>
<223>  Synthetic fragment derived from chlorotoxin (Leiurus
        quinquestiratus)

<400>  96

Lys Ser Tyr Gly Pro Gln Ser Leu Ser Arg
1               5                   10


<210>  97
<211>  10
<212>  PRT
<213>  Artificial Sequence

<220>
<223>  Synthetic fragment derived from chlorotoxin (Leiurus
        quinquestiratus)

<400>  97

Gly Lys Ser Tyr Gly Pro Gln Ser Leu Ser
1               5                   10


<210>  98
<211>  10
<212>  PRT
<213>  Artificial Sequence

<220>
<223>  Synthetic fragment derived from chlorotoxin (Leiurus
        quinquestiratus)

<400>  98

Arg Gly Lys Ser Tyr Gly Pro Gln Ser Leu
1               5                   10


<210>  99
<211>  10
<212>  PRT
<213>  Artificial Sequence

<220>
<223>  Synthetic fragment derived from chlorotoxin (Leiurus
       quinquestiratus)

<400>  99

Gly Arg Gly Lys Ser Tyr Gly Pro Gln Ser
1               5                   10


<210>  100
<211>  10
<212>  PRT
<213>  Artificial Sequence

<220>
<223>  Synthetic fragment derived from chlorotoxin (Leiurus
       quinquestiratus)

<400>  100

Lys Gly Arg Gly Lys Ser Tyr Gly Pro Gln
1               5                   10


<210>  101
<211>  10
<212>  PRT
<213>  Artificial Sequence

<220>
<223>  Synthetic fragment derived from chlorotoxin (Leiurus
       quinquestiratus)

<400>  101

Gly Lys Gly Arg Gly Lys Ser Tyr Gly Pro
1               5                   10


<210>  102
<211>  10
<212>  PRT
<213>  Artificial Sequence

<220>
<223>  Synthetic fragment derived from chlorotoxin (Leiurus
       quinquestiratus)

<400>  102

Gly Gly Lys Gly Arg Gly Lys Ser Tyr Gly
1               5                   10


<210>  103
<211>  10
<212>  PRT

<213>  Artificial Sequence

<220>
<223>  Synthetic fragment derived from chlorotoxin (Leiurus
       quinquestiratus)

<400>  103

Ser Gly Gly Lys Gly Arg Gly Lys Ser Tyr
1               5                   10


<210>  104
<211>  10
<212>  PRT
<213>  Artificial Sequence

<220>
<223>  Synthetic fragment derived from chlorotoxin (Leiurus
       quinquestiratus)

<400>  104

Ser Ser Gly Gly Lys Gly Arg Gly Lys Ser
1               5                   10


<210>  105
<211>  10
<212>  PRT
<213>  Artificial Sequence

<220>
<223>  Synthetic fragment derived from chlorotoxin (Leiurus
       quinquestiratus)

<400>  105

Asp Ser Ser Gly Gly Lys Gly Arg Gly Lys
1               5                   10


<210>  106
<211>  10
<212>  PRT
<213>  Artificial Sequence

<220>
<223>  Synthetic fragment derived from chlorotoxin (Leiurus
       quinquestiratus)

<400>  106

Asp Asp Ser Ser Gly Gly Lys Gly Arg Gly
1               5                   10


<210>  107
<211>  10
<212>  PRT
<213>  Artificial Sequence

<220>
<223>  Synthetic fragment derived from chlorotoxin (Leiurus
       quinquestiratus)

<400>  107

Ser Asp Asp Ser Ser Gly Gly Lys Gly Arg
1               5                   10


<210>  108
<211>  10
<212>  PRT
<213>  Artificial Sequence

<220>
<223>  Synthetic fragment derived from chlorotoxin (Leiurus
       quinquestiratus)

<400>  108

Lys Ser Asp Asp Ser Ser Gly Gly Lys Gly
1               5                   10


<210>  109
<211>  10
<212>  PRT
<213>  Artificial Sequence

<220>
<223>  Synthetic fragment derived from chlorotoxin (Leiurus
       quinquestiratus)

<400>  109

Arg Lys Ser Asp Asp Ser Ser Gly Gly Lys
1               5                   10


<210>  110
<211>  10
<212>  PRT
<213>  Artificial Sequence

<220>
<223>  Synthetic fragment derived from chlorotoxin (Leiurus
       quinquestiratus)

<400>  110

Ala Arg Lys Ser Asp Asp Ser Ser Gly Gly
1               5                   10


<210>  111
<211>  10
<212>  PRT
<213>  Artificial Sequence

<220>
<223>  Synthetic fragment derived from chlorotoxin (Leiurus
       quinquestiratus)

<400>  111

Met Ala Arg Lys Ser Asp Asp Ser Ser Gly
1               5                   10


<210>  112
<211>  10
<212>  PRT
<213>  Artificial Sequence

<220>
<223>  Synthetic fragment derived from chlorotoxin (Leiurus
      quinquestiratus)

<400>  112

Gln Met Ala Arg Lys Ser Asp Asp Ser Ser
1               5                   10


<210>  113
<211>  10
<212>  PRT
<213>  Artificial Sequence

<220>
<223>  Synthetic fragment derived from chlorotoxin (Leiurus
      quinquestiratus)

<400>  113

His Gln Met Ala Arg Lys Ser Asp Asp Ser
1               5                   10


<210>  114
<211>  10
<212>  PRT
<213>  Artificial Sequence

<220>
<223>  Synthetic fragment derived from chlorotoxin (Leiurus
      quinquestiratus)

<400>  114

Asp His Gln Met Ala Arg Lys Ser Asp Asp
1               5                   10


<210>  115
<211>  10
<212>  PRT
<213>  Artificial Sequence

<220>
<223>  Synthetic fragment derived from chlorotoxin (Leiurus
      quinquestiratus)

<400>  115

Thr Asp His Gln Met Ala Arg Lys Ser Asp
1               5                   10


<210>  116
<211>  10
<212>  PRT
<213>  Artificial Sequence

<220>
<223>  Synthetic fragment derived from chlorotoxin (Leiurus
      quinquestiratus)

<400>  116

Phe Thr Thr Asp His Gln Met Ala Arg Lys

1                   5                   10


<210>  117
<211>  10
<212>  PRT
<213>  Artificial Sequence

<220>
<223>  Synthetic fragment derived from chlorotoxin (Leiurus
       quinquestiratus)

<400>  117

Ser Phe Thr Thr Asp His Gln Met Ala Arg
1               5                   10


<210>  118
<211>  10
<212>  PRT
<213>  Artificial Sequence

<220>
<223>  Synthetic fragment derived from chlorotoxin (Leiurus
       quinquestiratus)

<400>  118

Pro Ser Phe Thr Thr Asp His Gln Met Ala
1               5                   10


<210>  119
<211>  10
<212>  PRT
<213>  Artificial Sequence

<220>
<223>  Synthetic fragment derived from chlorotoxin (Leiurus
       quinquestiratus)

<400>  119

Met Pro Ser Phe Thr Thr Asp His Gln Met
1               5                   10


<210>  120
<211>  10
<212>  PRT
<213>  Artificial Sequence

<220>
<223>  Synthetic fragment derived from chlorotoxin (Leiurus
       quinquestiratus)

<400>  120

Ser Met Pro Ser Phe Thr Thr Asp His Gln
1               5                   10


<210>  121
<211>  10
<212>  PRT
<213>  Artificial Sequence

<220>
<223>  Synthetic fragment derived from chlorotoxin (Leiurus
       quinquestiratus)

<400>  121

Met Ser Met Pro Ser Phe Thr Thr Asp His
1               5                   10